# EUROPÄISCHE PATENTSCHRIFT

(11) **EP 2 505 160 B1**
(45) Veröffentlichungstag und Bekanntmachung des Hinweises auf die Patenterteilung: **03.10.2018**
(21) Anmeldenummer: 12001821.3
(22) Anmeldetag: 14.03.2012
(51) Int. Cl.: A61B 90/50, F16M 11/10, G03B 21/14, G03B 21/16, F16M 11/18, F16M 11/20, F16M 11/38, F16M 11/42, G03B 21/20, G03B 21/56, G03B 21/58, A61B 90/00

(54) **Tragesystem für einen Operationssaal**
Carrying system for an operating theatre
Système de support pour une salle d'opération

(30) Priorität: 24.05.2011 DE 102011076322; 30.03.2011 DE 102011001681
(43) Veröffentlichungstag der Anmeldung: 03.10.2012
(73) Patentinhaber: Karl Storz SE & Co. KG, 78532 Tuttlingen (DE)
(72) Erfinder: Abri, Omid, 14129 Berlin (DE); Schrader, Stephan, 14532 Kleinmachnow (DE); Karge, Thorsten, 12163 Berlin (DE); Verkin, Julian, 16540 Hohen Neuendorf (DE)

(56) Entgegenhaltungen:
- EP-A1- 0 300 316
- EP-A1- 2 283 790
- CN-Y- 201 248 169
- DE-A1- 19 719 349
- DE-U1- 20 001 134
- DE-U1- 20 219 100
- DE-U1-202009 014 932
- US-A1- 2006 010 799

## Beschreibung

Die vorliegende Erfindung bezieht sich auf ein Tragesystem für einen Operationssaal oder einen anderen medizinischen Behandlungsraum.

Die Anzahl der Geräte und Systeme, die in einem modernen Operationssaal oder einem anderen medizinischen Behandlungsraum für diagnostische oder therapeutische Zwecke bereitgestellt werden, ist in der Vergangenheit immer weiter gestiegen. Die von diesen Geräten und Systemen bereitgestellten Funktionen nehmen zu, und die Menge der Chirurgen und anderem medizinischen Personal bereitgestellten und von diesen zu verarbeitenden Information wächst. Es gibt deshalb Bestrebungen, Funktionen, die bislang von zahlreichen separaten Geräten erfüllt wurden, in weniger Geräte und Systeme zu integrieren; die Anordnung der Geräte und Systeme zu verbessern; Information, die bislang an unterschiedlichen Orten bereitgestellt wurde, an einem gemeinsamen Ort und möglichst situationsabhängig aufbereitet darzustellen, insbesondere auf immer größeren Bildschirmen; und allgemein die Ergonomie zu verbessern.

In der US 3,931,452 ist eine Befestigung von Geräten an einer Decke eines Operationssaals beschrieben. Die feststehenden Enden von Tragarmen können an in der Decke verlaufende Schienen geschraubt werden, die auch Leitungen aufnehmenen können.

In der DE 299 03 798 U1 ist eine Anordnung zum Bewegen von Geräten im klinischen Bereich beschrieben. Ein angetriebener Mitnehmer läuft an einer an einer Wand befestigten Laufschiene entlang und zieht dabei ein mechanisch gekoppeltes Gerät, das mit eigenen Laufrollen auf dem Boden steht, beispielsweise aus einem Anästhesieraum in einen Operationssaal.

In der DE 197 19 349 A1 ist ein medizinischer Arbeitsplatz mit einem motorisch verstellbaren Projektor zum Projizieren von Bildern auf eine einrollbare Projektionsfläche beschrieben.

In der DE 200 01 134 U1 ist ein Operationssystem mit einem virtuellen Touchscreen, Gestikerkennung, einem Videoprojektor und einer Projektionsfläche beschrieben. Der Videoprojektor kann sich an einer Schieneneinrichtung befinden oder stationär befestigt sein. Der Videoprojektor und Displayflächen können motorisch verstellbar sein.

In der US2006/010799 ist Krankenhaus -Layouts und insbesondere das Layout, die Struktur und die Verwendung von Operationsräumen (OP) und verwandten Intubations-, Extubations- und Patientenzimmern beschrieben. In dieser Hinsicht ist ein Transportsystem zum Transportieren der Bewegung der mobilen Bildgebungsausrüstung zwischen den OP-/ Interventionsräumen vorgesehen. Zusätzlich kann das Transportsystem ein Überkopfschienensystem umfassen, das in die Decken der OP / Eingriffsräume eingebaut ist.

Eine Aufgabe der vorliegenden Erfindung besteht darin, eine verbesserte Ergonomie in einem medizinischen Behandlungsraum, insbesondere in einem Operationssaal, zu ermöglichen.

Diese Aufgabe wird durch den Gegenstand des unabhängigen Anspruchs gelöst.

Weiterbildungen sind in den abhängigen Ansprüchen angegeben.

Ausführungsformen der vorliegenden Erfindung beruhen auf der Idee, ein Tragesystem, insbesondere ein Anzeige- oder Deckenversorgungssystem, für einen Operationssaal mit einer oder mehreren gekrümmten Schienen, die an der Decke des Operationssaals angeordnet sind, und einer Hubeinrichtung, die entlang der Schienen bewegbar ist, zum vertikalen Bewegen einer Anzeigevorrichtung oder eines anderen medizinischen Geräts vorzusehen. Die Kombination einer oder mehrerer an der Decke des Operationssaals angeordneten Schienen mit einer Hubeinrichtung ermöglicht auch ohne die herkömmlichen, einige Nachteile aufweisende Tragarme, eine weitgehend beliebige Anordnung des medizinischen Geräts.

Ein Tragesystem für einen medizinischen Behandlungsraum umfasst eine an einer Decke des medizinischen Behandlungsraums zu befestigende Schieneneinrichtung, die einen gekrümmten Pfad vorgibt, und eine Hubeinrichtung zum Bewegen eines medizinischen Geräts zwischen einer oberen Ruheposition und einer unteren Betriebsposition, wobei die Hubeinrichtung entlang des von der Schieneneinrichtung vorgegebenen Pfads bewegbar ist.

Das Tragesystem ist insbesondere ein Anzeigesystem oder ein Deckenversorgungssystem oder Bestandteil eines Deckenversorgungssystems. Das Tragesystem ist insbesondere für einen Operationssaal vorgesehen und ausgebildet. Die Schieneneinrichtung umfasst eine oder mehrere-insbesondere parallele-Schienen oder in ihrer Funktion Schienen entsprechende Bauteile. Insbesondere umfasst die Schieneneinrichtung eine oder mehrere Laufflächen und/oder eine oder mehrere Gleitflächen für Räder bzw. Gleitbeläge an einer oder mehreren Laufkatzeneinrichtungen. Der von der Schieneneinrichtung vorgegebene gekrümmte Pfad weist insbesondere einen Krümmungsmittelpunkt auf, der über einem Operationstisch oder einem anderen für einen Patienten vorgesehenen Aufenthaltsort liegt.

Die Hubeinrichtung ist insbesondere zum vertikalen oder im Wesentlichen vertikalen Bewegen des medizinischen Geräts ausgebildet. Insbesondere ist die Bewegung zwischen der oberen Ruheposition und der unteren Betriebsposition im Wesentlichen vertikal, wenn der horizontale Abstand zwischen der oberen Ruheposition und der unteren Betriebsposition höchstens so groß (und insbesondere höchstens halb so groß) ist wie der vertikale Abstand der oberen Ruheposition und der unteren Betriebsposition. Die Hubeinrichtung kann jeweils eine oder mehrere Gasdruck- oder andere Federn, Elektromotoren, pneumatische oder hydraulische Aktoren oder andere Antriebseinrichtungen zum Bewegen des medizinischen Geräts in vertikaler Richtung umfassen. Die Hubeinrichtung kann ausgebildet sein, um das medizinische Gerät entlang eines vorbestimmten Hubpfads zu bewegen, der im Wesentlichen vertikal oder (im Rahmen der erwünschten und üblicherweise erzielten Präzision) genau vertikal verläuft.

Der durch die Bewegbarkeit der Hubeinrichtung entlang der Schieneneinrichtung bereitgestellte horizontale Freiheitsgrad und der durch die Hubeinrichtung bereitgestellte vertikale Freiheitsgrad können eine für viele Anwendungen ausreichende Bewegbarkeit eines medizinischen Geräts ermöglichen. Für diese Bewegbarkeit ist kein Tragarm erforderlich. Die herkömmlich wachsende Anzahl von um vertikale Achsen schwenkbaren Tragarmen zum Tragen bzw. Halten von Operationsleuchten, Bildschirmen und anderen Geräten bringt eine Reihe von Nachteilen mit sich. Insbesondere muss bei jedem Verschieben einer Operationsleuchte, eines Bildschirms oder eines anderen Geräts sichergestellt werden, dass sich die Tragarme nicht gegenseitig behindern, blockieren oder beschädigen. Ferner weisen herkömmlich verwendete Tragarme in der Regel massive rechteckige Querschnitte auf, die eine von der Decke des Behandlungsraums ausgehende Luftströmung (in Anlehnung an den englischen Begriff "laminar air flow" auch als LAF bezeichnet) behindern und verwirbeln. Eine laminare vertikale Strömung staub- und keimarmer Luft ist mit den herkömmlichen, immer zahlreicher werdenden Tragarmen immer weniger erzielbar. Bei einer Anordnung der Schieneneinrichtung außerhalb oder am Rand des laminaren Strömungsfelds kann das vorliegende Tragesystem eine weitgehende freie Bewegbarkeit des medizinischen Geräts ohne Störung des laminaren Strömungsfelds ermöglichen.

Bei einem Tragesystem, wie es hier beschrieben ist, ist das medizinische Gerät insbesondere eine Anzeigevorrichtung oder ein Teil einer Anzeigevorrichtung, wobei mittels der Anzeigevorrichtung in der unteren Betriebsposition Information für medizinisches Personal sichtbar dargestellt werden kann.

Die Anzeigevorrichtung ist insbesondere zum Anzeigen von unbewegten oder bewegten Bildern, Grafik, Text, Zahlen, Symbolen und von anderen Darstellungen von Information ausgebildet.

An der Schieneneinrichtung können, insbesondere mittels einer oder mehrerer weiterer Hubeinrichtungen, eine oder mehrere weitere Anzeigevorrichtungen oder andere medizinisehe Geräte bewegbar bzw. verschiebbar aufgehängt sein, wodurch die Anzahl der Tragarme deutlich vermindert oder auf die Verwendung von Tragarmen ganz verzichtet werden kann.

Bei einem Tragesystem mit einer Anzeigevorrichtung, wie es hier beschrieben ist, umfasst die Anzeigevorrichtung insbesondere eine Projektionsfläche, einen Projektor zum Projizieren einer Darstellung von Information auf die Projektionsfläche und eine Positionierungseinrichtung zur Festlegung der Positionen des Projektors und der Projektionsfläche, wobei die Positionierungseinrichtung ausgebildet ist, um den Projektor und die Projektionsfläche an im Wesentlichen gegenüberliegenden Positionen des von der Schieneneinrichtung vorgegebenen Pfads zu positionieren.

Die Positionierungseinrichtung umfasst insbesondere eine mechanische Kopplung. Beispielsweise verläuft ein Seil entlang des durch die Schieneneinrichtung vorgegebenen Pfads, wobei sowohl die Projektionsfläche als auch der Projektor an voneinander beabstandeten Stellen mit dem Seil gekoppelt sind. Alternativ umfasst die Positionierungseinrichtung beispielsweise eine Steuerung zum Steuern der Positionierung des Projektors und der Projektionsfläche, insbesondere über nachfolgend beschriebene Antriebseinrichtungen.

Der Projektor und die Projektionsfläche sind an im Wesentlichen gegenüberliegenden Positionen des von der Schieneneinrichtung vorgegebenen Pfads angeordnet, wenn der Winkelabstand (bezogen auf den Mittelpunkt der von dem vorgegebenen Pfad umschriebenen Fläche) zwischen dem Mittelpunkt der Lichtaustrittsfläche des Projektors und dem Mittelpunkt der Projektionsfläche mindestens 120 Grad beträgt. Der Projektor und die Projektionsfläche sind ferner an im Wesentlichen gegenüberliegenden Positionen des von der Schieneneinrichtung vorgegebenen Pfads angeordnet, wenn der entlang des vorgegebenen Pfads gemessene Abstand zwischen einander entsprechenden Punkten an einer Laufkatzeneinrichtung des Projektors und an einer Laufkatzeneinrichtung der Projektionsfläche mindestens ein Drittel der Gesamtlänge des vorgegebenen Pfads beträgt. Der Projektor und die Projektionsfläche sind ferner an im Wesentlichen gegenüberliegenden Positionen des von der Schieneneinrichtung vorgegebenen Pfads angeordnet, wenn der Abstand der Geraden durch den Mittelpunkt der Lichtaustrittsfläche des Projektors und den Mittelpunkt der Projektionsfläche von der Flächennormale auf den Mittelpunkt der durch den vorgegebenen Pfad umschriebenen Fläche höchstens ein Vierteil der Summe der Abstände der beiden Mittelpunkte der Lichtaustrittsfläche des Projektors und der Projektionsfläche von der Flächennormale entspricht.

Die Hubeinrichtung ist insbesondere ausgebildet, um die Projektionsfläche vertikal zu bewegen. Die Projektionsfläche kann zusätzlich faltbar oder klappbar sein, um in der oberen Ruheposition einen geringen Raumbedarf aufzuweisen.

Ein Tragesystem, wie es hier beschrieben ist, kann ferner eine Antriebsreinrichtung zum motorischen Bewegen des medizinischen Geräts (insbesondere der Anzeigevorrichtung oder eines Bestandteils der Anzeigevorrichtung) entlang des durch die Schieneneinrichtung vorgegebenen Pfads umfassen. Wenn das Tragesystem eine Anzeigevorrichtung in Gestalt eines Projektors und einer Projektionsfläche trägt, sind insbesondere je eine Antriebseinrichtung zum Bewegen des Projektors und der Projektionsfläche entlang des vorgegebenen Pfads vorgesehen.

Bei einem Tragesystem, wie es hier beschrieben ist, umfasst die Positionierungseinrichtung insbesondere eine Steuerung und zumindest entweder eine erste Antriebseinrichtung zum Bewegen des Projektors entlang des durch die Schieneneinrichtung vorgegebenen Pfads oder eine zweite Antriebseinrichtung zum Bewegen der Projektionsfläche entlang des durch die Schieneneinrichtung vorgegebenen Pfads, wobei die Steuerung einen Steuerausgang zum Steuern zumindest entweder der ersten Antriebseinrichtung oder der zweiten Antriebseinrichtung aufweist.

Weitere Antriebseinrichtungen können zum Heben und zum Schwenken des Projektors und/oder der Projektionsfläche in jeweils einer oder in zwei Richtungen vorgesehen sein.

Die Antriebseinrichtung oder die Antriebseinrichtungen können eine selbsttätige bzw. automatische Positionierung der Anzeigevorrichtung bzw. ihrer Bestandteile oder eine beispielsweise durch Gesten gesteuerte Positionierung, die keine Berührung der Anzeigevorrichtung oder ihrer Bestandteil erfordert, ermöglichen.

Ein Tragesystem mit einer Antriebseinrichtung zum motorischen Bewegen der Anzeigevorrichtung oder eines Bestandteils der Anzeigevorrichtung, wie es hier beschrieben ist, kann ferner eine Einrichtung zum Erfassen einer Position oder einer Geste eines medizinischen Personals und zum Steuern der Antriebseinrichtung in Abhängigkeit von der erfassten Position oder Geste umfassen.

Die Einrichtung zum Erfassen umfasst insbesondere eine Laufzeitkamera (englisch: time of flight camera), eine Stereokamera oder eine oder mehrere andere Kameras zum zweidimensionalen oder dreidimensionalen Erfassen eines Raumbereichs, wobei der Raumbereich insbesondere das Operationsgebiet, den Operationstisch und/oder dessen Umgebung umfasst. Die Einrichtung zum Erfassen umfasst ferner eine Bilderkennung bzw. Mustererkennung zur Identifizierung von medizinischem Personal oder des Kopfs oder einer Hand oder eines anderen Körperteils des medizinischen Personals.

Eine Einrichtung zum Erfassen einer Position oder einer Geste kann eine selbsttätige bzw. automatische Anpassung der Positionen der Anzeigevorrichtung bzw. des Projektors und der Projektionsfläche an den momentanen Aufenthaltsort des relevanten medizinischen Personals und an die momentane Blickrichtung ermöglichen. Ferner können die Positionen weiterer Personen und von Geräten berücksichtigt werden, um eine Abschattung oder teilweise Bedeckung der Anzeigevorrichtung zu verhindern und eine uneingeschränkte Ablesbarkeit zu gewährleisten. Eine Erfassung von Gesten kann eine einfache und intuitive Steuerung des Tragesystems ermöglichen. Medizinisches Personal kann dadurch entlastet werden und sich in stärkerem Maße auf seine eigentlichen medizinischen Aufgaben konzentrieren.

Bei einem Tragesystem, bei dem die Positionierungseinrichtung Antriebseinrichtungen und eine Steuerung umfasst, weist die Steuerung insbesondere einen Eingang zum Empfangen eines Positionssignals, das von der Position der Projektionsfläche relativ zum Projektor oder von den absoluten Positionen von Projektionsfläche und Projektor abhängig ist, auf. Alternativ zu einer Erfassung der absoluten oder relativen Positionen des Projektors und der Projektionsfläche können die Antriebseinrichtungen Schrittmotoren aufweisen, wobei die Positionen durch Zählung bzw. Registrierung der zurückgelegten Schritte bestimmt werden können.

Bei einem Tragesystem, wie es hier beschrieben ist, weist der von der Schieneneinrichtung vorgegebene Pfad insbesondere die Gestalt eines Teils einer Randlinie oder einer vollständigen Randlinie einer Ellipse oder eines Kreises oder eines Rechtecks mit abgerundeten Ecken oder einer Parabel oder einer Hyperbel auf.

Der von der Schieneneinrichtung vorgegebene Pfad kann offen (mit zwei Enden) oder geschlossen sein und die Gestalt eines Rands einer Ellipse oder eines Kreises oder eines Abschnitts eines solchen Rands aufweisen. Der von der Schieneneinrichtung vorgegebene Pfad ist insbesondere in einem medizinischen Behandlungsraum so anzuordnen, dass ein Operationstisch oder ein anderer für den Aufenthalt eines Patienten während eines diagnostischen, therapeutischen oder chirurgischen Verfahrens vorgesehene Ort in der Mitte der von dem Pfad umschriebenen Fläche oder in der Nähe dieser Mitte angeordnet ist. Diese relative Anordnung des Operationstisches und des durch die Schieneneinrichtung vorgegebenen Pfads kann es ermöglichen, ein medizinisches Gerät, insbesondere eine Anzeigevorrichtung, unabhängig von dem Ort, an dem eine Ärztin oder ein Arzt oder anderes relevantes medizinisches Personal am Operationstisch steht, und weitgehend unabhängig von der Blickrichtung des medizinischen Personals ergonomisch vorteilhaft oder optimal anzuordnen. Eine vorteilhafte Anordnung des medizinischen Geräts kann dadurch weiter erleichtert werden, dass das medizinische Gerät um eine vertikale Achse und/oder um eine horizontale Achse schwenkbar an der Hubeinrichtung angeordnet ist.

Ein Tragesystem, wie es hier beschrieben ist, kann ferner eine Übertragungseinrichtung zum elektrischen Übertragen zumindest entweder von elektrischer Leistung oder eines elektrischen Signals umfassen, wobei die Übertragungseinrichtung eine starr angeordnete Kontaktschiene und eine mit der Hubeinrichtung mechanisch gekoppelte und bewegbare Kontakteinrichtung, die mit der Kontaktschiene einen mechanischen und elektrisch leitfähigen Kontakt bildet, aufweist.

Die Kontaktschiene oder mehrere Kontaktschienen sind insbesondere parallel zu einer bzw. mehreren Schienen der Schieneneinrichtung angeordnet oder mit diesen identisch. Die mit der Anzeigevorrichtung bzw. einem Bestandteil der Anzeigevorrichtung mechanisch gekoppelte und bewegbare Kontakteinrichtung umfasst beispielsweise eine Kontaktrolle oder ein Kontakt-Schleifstück aus einem elektrisch leitfähigen Material. Die durch die Übertragungseinrichtung übertragbaren elektrischen Signale umfassen insbesondere ein Positionssignal, wie es oben beschrieben ist, und/oder ein Steuersignal zum Steuern der Position der Anzeigevorrichtung entlang des vorgegebenen Pfads.

Ein Tragesystem, wie es hier beschrieben ist, kann ferner eine Tragvorrichtung zum Halten eines weiteren medizinischen Geräts umfassen, wobei die Tragvorrichtung entlang des von der Schieneneinrichtung vorgegebenen Pfads bewegbar ist.

Die Schieneneinrichtung kann somit neben einer horizontalen Bewegbarkeit von einer oder mehreren Anzeigevorrichtungen eine entsprechende horizontale Bewegbarkeit (weiterer) medizinischer bzw. für medizinische Verwendung vorgesehener Geräte ermöglichen. Insbesondere können alle oder fast alle in einem medizinischen Behandlungsraum bewegbar anzuordnende Geräte durch Tragvorrichtungen gehalten sein, wobei sie entlang des durch die Schieneneinrichtung vorgegebenen Pfads bewegbar sind. Die Anzahl der erforderlichen herkömmlichen Tragarme kann dadurch reduziert werden, unter Umständen kann auf Tragarme ganz verzichtet werden.

Bei einem Tragesystem mit einer Tragvorrichtung zum Halten eines medizinischen Geräts, wie es hier beschrieben ist, kann die Schieneneinrichtung mehrere Schienen umfassen, wobei die Tragvorrichtung an einer Schiene hängt, an der die Anzeigeeinrichtung nicht hängt.

Die Schienen der Schieneneinrichtung sind insbesondere parallel oder im Wesentlich parallel zueinander angeordnet. Alle Schienen der Schieneneinrichtung können in einer Ebene oder im Wesentlichen in einer Ebene angeordnet sein. Alternativ können die Schienen der Schieneneinrichtung in verschiedenen (insbesondere parallelen) Ebenen angeordnet sein.

Eine Schieneneinrichtung mit mehreren Schienen kann eine unabhängige Bewegbarkeit der Anzeigevorrichtung und anderer Geräte entlang des oder der von der Schieneneinrichtung vorgegebenen Pfade ermöglichen. Dies kann die Flexibilität in der Anordnung der Geräte innerhalb des medizinischen Behandlungsraums weiter verbessern.

Ein Tragesystem mit einer Tragvorrichtung zum Halten eines medizinischen Geräts, wie es hier beschrieben ist, kann ferner eine Antriebseinrichtung zum motorischen Bewegen der Tragvorrichtung entlang des von der Schieneneinrichtung vorgegebenen Pfads umfassen.

Insbesondere wenn die Antriebseinrichtungen durch eine Steuerung gesteuert werden, die mittels einer Kamera oder anderer Sensoren die Positionen von medizinischem Personal und/oder der Anzeigevorrichtung und der Tragvorrichtung erfasst, kann eine teilweise oder vollständig selbstständige Bewegung der Anzeigevorrichtung und anderer Geräte im medizinischen Behandlungsraum erfolgen, um das medizinische Personal zu entlasten.

Ein Tragesystem, wie es hier beschrieben ist, kann ferner einen Versorgungsarm umfassen, der um einen durch ein Gelenk vorbestimmten Ort rotierbar ist, wobei der Versorgungsarm an einem von dem Gelenk beabstandeten Ort mit der Hubeinrichtung oder mit der Tragvorrichtung gekoppelt ist, und wobei der Versorgungsarm zur Übertragung zumindest entweder eines Signals oder von Leistung oder eines Fluids ausgebildet ist.

Der Versorgungsarm kann, insbesondere bei Kreisform des durch die Schieneneinrichtung vorgegebenen Pfads, starr ausgebildet sein. Alternativ kann der Versorgungsarm biegeflexibel oder teleskopierbar ausgebildet sein. Eine teleskopierbare Ausbildung des Versorgungsarms ermöglicht insbesondere eine elliptische oder in anderer Weise von einer Kreisform abweichende Gestalt des durch die Schieneneinrichtung vorgegebenen Pfads.

Der durch das Gelenk vorbestimmte Ort ist insbesondere näherungsweise in der Mitte der durch den vorbestimmten Pfad umschriebenen Fläche angeordnet. Aufgrund des Gelenks nahe der Mitte der durch den vorgegebenen Pfad umschriebenen Fläche ist der Versorgungsarm insbesondere um eine vertikale oder im Wesentlichen vertikale Achse rotierbar bzw. schwenkbar. Zusätzlich kann das Gelenk eine Schwenkbarkeit um eine horizontale Achse ermöglichen.

Der Versorgungsarm umfasst insbesondere ein erstes bzw. radial inneres Ende, das das Gelenk aufweist, und ein zweites bzw. radial äußeres Ende, das mit der Hubeinrichtung bzw. mit der Tragvorrichtung gekoppelt ist. Eine gelenkige Kopplung des radial äußeren Endes des Versorgungsarms mit der Hubeinrichtung bzw. mit der Tragvorrichtung und eine Verschiebbarkeit des radial äußeren Endes des Versorgungsarms relativ zu der Hubeinrichtung bzw. zu der Tragvorrichtung kann eine Verwendbarkeit des Versorgungsarms bei einem durch die Schieneneinrichtung vorgegebenen Pfad, der nicht kreisförmig ist, ermöglichen.

Der Versorgungsarm weist insbesondere jeweils eine oder mehrere elektrische Leitungen und/oder Lichtleitkabel und/oder Rohre und/oder Schläuche auf. Während elektrische Leistung und elektrische Signale mit einer niedrigen Datenrate beispielsweise mit der oben beschriebenen Übertragungseinrichtung an die Hubeinrichtung oder an die Tragvorrichtung übertragen werden können, gilt dies für optische Signale und für elektrische Signale mit hohen Datenübertragungsraten nur eingeschränkt und für Fluide gar nicht. Der Versorgungsarm schafft eine Möglichkeit zuverlässig, unterbrechungsfrei und unabhängig von der Position der Hubeinrichtung bzw. der Tragvorrichtung entlang des durch die Schieneneinrichtung vorgegebenen Pfads nicht nur elektrische Leistung, sondern auch elektrische und optische Signale und Fluide zu oder von einer an der Hubeinrichtung angeordneten Anzeigevorrichtung bzw. einem von der Tragvorrichtung gehaltenen medizinischen Gerät zu übertragen.

Dazu weist der Versorgungsarm insbesondere am Gelenk oder in das Gelenk teilweise oder vollständig integriert eine Drehdurchführung oder eine andere Einrichtung zur kontinuierlichen und unterbrechungsfreien elektrischen oder optischen Übertragung von Leistung und/oder Signalen und/oder zur Übertragung von einem oder mehreren Fluiden bzw. Medien auf. Die Drehdurchführung oder andere Einrichtung ist ausgebildet, um unabhängig von der Orientierung bzw. Position des Versorgungsarms und insbesondere auch während einer Bewegung bzw. Rotation des Versorgungsarms um das Gelenk und insbesondere auch nach einer Rotation um einen beliebigen Winkel eine Übertragung von Leistung, Signalen und/oder Fluiden bzw. Medien zu ermöglichen.

Die Drehdurchführung oder die andere Einrichtung kann eine oder mehrere Dichtungseinrichtungen aufweisen. Als Dichtungseinrichtungen kommen insbesondere Radial-Wellendichtungen (oft auch als Simmerringe bezeichnet), Axial-Wellendichtungen oder anderer Dichtungseinrichtungen, die eine druckdichte Abdichtung gegen den Übertritt eines Fluids ermöglichen, in Betracht. Zu Übertragung elektrischer Leistung oder elektrischer Signale können Schleifringe und Gleit- bzw. Schleifkontakte vorgesehen sein.

Alternativ sind verdrillbare bzw. verwindbare oder spiralförmig auf- und abwickelbare elastische elektrische, optische oder Fluidleitungen verwendbar, die keine beliebige sondern nur eine auf ein Winkelintervall begrenzte Rotation ermöglichen. Das Winkelintervall kann mehr als 360 Grad umfassen.

Der Versorgungsarm ist insbesondere nicht zur Aufnahme der Gewichtskraft der Hubeinrichtung oder der Tragvorrichtung oder eines medizinischen Geräts ausgebildet.

Der Versorgungsarm stellt also keinen Tragarm dar, wie er herkömmlich vielfach zum Tragen von Operationsleuchten, Bildschirmen und anderen Geräten in medizinischen Behandlungsräumen verwendet wird. Der Versorgungsarm kann deshalb für deutlich geringere mechanische Belastungen ausgelegt sein und einen deutlich kleineren Querschnitt aufweisen als ein herkömmlicher Tragarm. Ferner kann der Querschnitt des Versorgungsarms so ausgebildet sein, dass der Versorgungsarm ein vertikales laminares Strömungsfeld nicht oder nur geringfügig stört. Insbesondere weist der Versorgungsarm einen Querschnitt auf, dessen Breite kleiner als (insbesondere nur halb so groß wie) die Höhe ist, wobei die Breite in horizontaler Richtung und die Höhe in vertikaler Richtung gemessen werden. Ferner kann der Versorgungsarm einen tropfenförmigen Querschnitt aufweisen, um den Einfluss des Versorgungsarms auf das vertikale laminare Strömungsfeld zu vermindern.

Eine Tragsystem, wie sie hier beschrieben ist, kann ferner eine Führungseinrichtung zum Führen des medizinischen Gerätes entlang eines vorbestimmten Pfads zwischen der Ruheposition und der Betriebsposition aufweisen.

Der vorbestimmte Pfad ist insbesondere gerade und vertikal oder im Wesentlichen vertikal. Die Führungseinrichtung kann mit der Hubeinrichtung teilweise oder vollständig integriert sein.

Die Führung des medizinischen Geräts entlang des vorbestimmten Pfads kann die Betriebssicherheit der Tragvorrichtung verbessern. Ferner kann die Führungseinrichtung die untere Betriebsposition des medizinischen Gerätes in lateraler Richtung definieren und damit die Verwendung des medizinischen Geräts in der Betriebsposition vereinfachen.

Bei einem Tragesystem, wie es hier beschrieben ist, umfasst die Führungseinrichtung insbesondere eine Scherenmechanik, die ausgebildet ist, um das medizinische Gerät unabhängig von seiner Position in einer vorbestimmten Orientierung zu halten.

Eine Scherenmechanik aus einer Mehrzahl von paarweise gekreuzten und an ihren Kreuzungspunkten und ihren Enden gelenkig miteinander verbundenen Holmen kann eine definierte Parallelverschiebung des medizinischen Geräts ermöglichen. Insbesondere ist die Scherenmechanik ausgebildet, um das medizinische Gerät sowohl in seiner oberen Ruheposition als auch in seiner unteren Betriebsposition waagrecht zu halten.

Ferner kann eine Scherenmechanik Teil einer Hubeinrichtung sein, wobei zum vertikalen Bewegen des Gerätehalters beispielsweise eine Ausübung einer Kraft auf das oberste Paar von Holmen relativ zueinander ausreicht.

Bei einem Tragesystem, wie es hier beschrieben ist, kann die Führungseinrichtung eine teleskopierbare Einrichtung umfassen.

Eine teleskopierbare Einrichtung umfasst insbesondere eine oder mehrere in Serie geschaltete Linearführungen.

Ein Tragesystem, wie es hier beschrieben ist, kann ferner ein Seil und eine Antriebseinrichtung zum Bewegen des medizinischen Geräts zwischen der Ruheposition und der Betriebsposition mittels des Seils umfassen.

Die Antriebseinrichtung und das Seil sind insbesondere Bestandteile der Hubeinrichtung oder bilden die Hubeinrichtung. Die Antriebseinrichtung umfasst beispielsweise einen elektrischen oder pneumatischen oder hydraulischen Motor mit einer Haspel zum Aufwickeln des Seils. Mehrere Seile, die mittels derselben Haspel oder mittels mehrerer mechanisch miteinander gekoppelter Haspeln gleichzeitig bewegt werden, können in jeder Position des Gerätehalters eine erwünschte Orientierung desselben gewährleisten.

Die vorliegende Erfindung beruht ferner auf der Idee, in einem medizinischen Behandlungsraum unter dessen Deckenfläche einen Versorgungsarm vorzusehen, der um einen durch ein Gelenk vorbestimmten Ort rotierbar ist, wobei der Versorgungsarm ausgebildet ist, um zumindest entweder ein Signal oder Leistung oder ein Fluid zwischen dem Gelenk und einem von dem Gelenk beabstandeten Ort zu übertragen.

Der Versorgungsarm ist insbesondere ausgebildet, um elektrische und/oder optische Signale und/oder elektrische Leistung und/oder Licht einer Lichtquelle (beispielsweise für Endoskopie) und/oder Druckluft oder ein anderes Fluid von dem ortsfest angeordneten Gelenk zu einem zumindest auf einem Umfang eines Kreises um das Gelenk einstellbaren Ort zu übertragen. Ferner kann der Versorgungsarm ausgebildet sein, um ein Signal und/oder ein Fluid in umgekehrter Richtung zu leiten bzw. zu übertragen. Dazu weist der Versorgungsarm insbesondere jeweils eine oder mehrere elektrische Leitungen und/oder Lichtleitkabel und/oder Rohre und/oder Schläuche auf.

Dazu weist der Versorgungsarm insbesondere am Gelenk oder in das Gelenk teilweise oder vollständig integriert eine Drehdurchführung oder eine andere Einrichtung zur kontinuierlichen und unterbrechungsfreien elektrischen oder optischen Übertragung von Leistung und/oder Signalen und/oder zur Übertragung von einem oder mehreren Fluiden bzw. Medien auf. Die Drehdurchführung oder andere Einrichtung ist ausgebildet, um unabhängig von der Orientierung bzw. Position des Versorgungsarms und insbesondere auch während einer Bewegung bzw. Rotation des Versorgungsarms um das Gelenk und insbesondere auch nach einer Rotation um einen beliebigen Winkel eine Übertragung von Leistung, Signalen und/oder Fluiden bzw. Medien zu ermöglichen.

Die Drehdurchführung oder die andere Einrichtung kann eine oder mehrere Dichtungseinrichtungen aufweisen. Als Dichtungseinrichtungen kommen insbesondere Radial-Wellendichtungen (oft auch als Simmerringe bezeichnet), Axial-Wellendichtungen oder anderer Dichtungseinrichtungen, die eine druckdichte Abdichtung gegen den Übertritt eines Fluids ermöglichen, in Betracht. Zu Übertragung elektrischer Leistung oder elektrischer Signale können Schleifringe und Gleit- bzw. Schleifkontakte vorgesehen sein.

Alternativ sind verdrillbare bzw. verwindbare oder spiralförmig auf- und abwickelbare elastische elektrische, optische oder Fluidleitungen verwendbar, die keine beliebige sondern nur eine auf ein Winkelintervall begrenzte Rotation ermöglichen. Das Winkelintervall kann mehr als 360 Grad umfassen.

Der Versorgungsarm ist insbesondere ausgebildet, um ein in einem Operationssaal bewegbares medizinisches Gerät mit den Signalen, Leistung oder einem Fluid zu versorgen und/oder Signale oder ein Fluid von dem medizinischen Gerät zu dem Gelenk zu übertragen. Zwischen dem Gelenk und entsprechenden Signal-, Leistungs- oder Fluidquellen oder -senken sind entsprechende Leitungen vorgesehen, die insbesondere ortsfest und insbesondere über der Deckenfläche des medizinischen Behandlungsraums verlaufen.

Der Versorgungsarm kann insbesondere starr ausgebildet sein, wodurch eine Versorgung eines Geräts ermöglicht wird, das sich in einem kreisringförmigen Raumbereich befindet. Alternativ kann der Versorgungsarm biegeflexibel oder teleskopierbar ausgebildet sein. Eine teleskopierbare Ausbildung des Versorgungsarms ermöglicht eine Versorgung medizinischer Geräte, die sich in einem größeren Raumbereich befinden.

Der Versorgungsarm ist insbesondere nicht zur Aufnahme der Gewichtskraft eines Geräts oder einer anderen Einrichtung ausgebildet. Der Versorgungsarm stellt also keinen Tragarm dar, wie er herkömmlich vielfach zum Tragen von Operationsleuchten, Bildschirmen und anderen Geräten in medizinischen Behandlungsräumen verwendet wird. Der Versorgungsarm kann deshalb für deutlich geringere mechanische Belastungen ausgelegt sein und einen deutlich kleineren Querschnitt aufweisen als ein herkömmlicher Tragarm. Ferner kann der Querschnitt des Versorgungsarms so ausgebildet sein, dass der Versorgungsarm ein vertikales laminares Strömungsfeld nicht oder nur geringfügig stört. Insbesondere weist der Versorgungsarm einen Querschnitt auf, dessen Breite kleiner als (insbesondere nur halb so groß wie) die Höhe ist, wobei die Breite in horizontaler Richtung und die Höhe in vertikaler Richtung gemessen werden. Ferner kann der Versorgungsarm einen tropfenförmigen Querschnitt aufweisen, um den Einfluss des Versorgungsarms auf das vertikale laminare Strömungsfeld zu vermindern.

### Kurzbeschreibung der Figuren

Nachfolgend werden Ausführungsformen anhand der beigefügten Figuren näher erläutert. Es zeigen:
- Figur 1: eine schematische Darstellung eines medizinischen Behandlungsraums;
- Figur 2: eine weitere schematische Darstellung des medizinischen Behandlungsraums aus Figur 1;
- Figur 3: eine schematische Darstellung eines weiteren medizinischen Behandlungsraums;
- Figur 4: eine schematische Schnittdarstellung einer Schieneneinrichtung;
- Figur 5: eine schematische Schnittdarstellung einer weiteren Schieneneinrichtung;
- Figur 6: eine schematische Schnittdarstellung einer weiteren Schieneneinrichtung;
- Figur 7: eine schematische Schnittdarstellung einer weiteren Schieneneinrichtung;
- Figur 8: eine schematische Schnittdarstellung einer weiteren Schieneneinrichtung;
- Figur 9: eine schematische Schnittdarstellung einer weiteren Schieneneinrichtung;
- Figur 10: eine schematische Darstellung einer Tragvorrichtung;
- Figur 11: eine schematische Darstellung einer weiteren Tragvorrichtung;
- Figur 12: eine schematische Darstellung eines erfindungsgemäßen Versorgungsarms;
- Figur 13: eine schematische Darstellung einer Anzeigevorrichtung;
- Figur 14: eine schematische Darstellung einer weiteren Tragvorrichtung;
- Figur 15: eine schematische Darstellung einer Projektionsfläche;
- Figur 16: eine weitere schematische Darstellung der Projektionsfläche aus Figur 15;
- Figur 17: eine weitere schematische Darstellung der Projektionsfläche aus den Figuren 15 und 16;
- Figur 18: eine schematische Darstellung einer weiteren Projektionsfläche;
- Figur 19: eine weitere schematische Darstellung der Projektionsfläche aus Figur 18;
- Figur 20: eine schematische Darstellung einer weiteren Projektionsfläche;
- Figur 21: eine schematische Darstellung einer weiteren Projektionsfläche;
- Figur 22: eine weitere schematische Darstellung der Projektionsfläche aus den Figuren 20 und 21;
- Figur 23: eine schematische Darstellung einer weiteren Projektionsfläche;
- Figur 24: eine schematische Darstellung einer weiteren Projektionsfläche;
- Figur 25: eine weitere schematische Darstellung der Projektionsfläche aus Figur 24;
- Figur 26: eine schematische Darstellung eines Projektionssystems;
- Figur 27: eine schematische Darstellung eines weiteren Projektionssystems;
- Figur 28: eine schematische Darstellung einer Steuerung.

### Beschreibung der Ausführungsformen

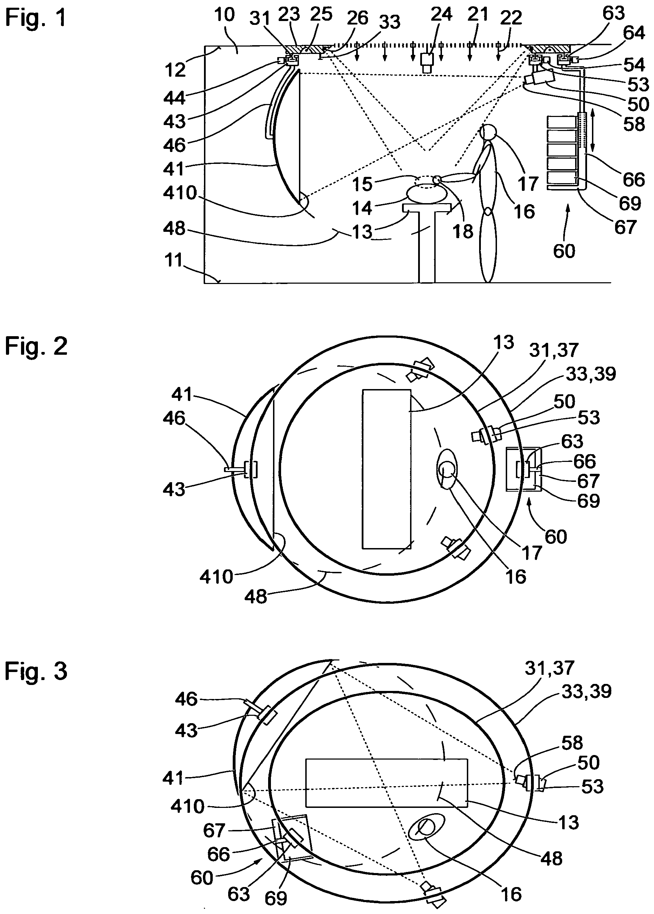

Figur 1 zeigt eine schematische Darstellung eines medizinischen Behandlungsraums 10, insbesondere eines Operationssaals, mit einer Bodenfläche 11 und einer Deckenfläche 12. Die Zeichenebene der Figur 1 ist senkrecht zu der Bodenfläche 11 und zu der Deckenfläche 12. Die Deckenfläche 12 ist insbesondere eben oder im Wesentlichen eben und wird durch die aus dem Inneren des medizinischen Behandlungsraums 10 sichtbare Unterseite der Decke gebildet. Auf der Bodenfläche 11 des medizinischen Behandlungsraums 10 steht oder ist befestigt ein Behandlungstisch 13, auf dem ein Patient 14 beispielsweise während einer Operation liegen kann. Durch eine gestrichelte Linie ist die Ausdehnung eines Operationsfelds 15 angedeutet. Neben dem Behandlungstisch 13 steht eine Ärztin, ein Arzt oder anderes medizinisches Personal 16 mit einem Kopf 17. Das medizinische Personal 16 hält eine Hand 18 im Operationsfeld 15.

In einem mittleren Bereich über dem Behandlungstisch 13 sind zahlreiche Luftdüsen 21 an der Deckenfläche 12 angeordnet. Die Luftdüsen 21 werden beispielsweise durch Öffnungen in Gittern gebildet. Die Luftdüsen 21 sind ausgebildet, um eine vertikales laminares Strömungsfeld 22 staub- und keimarmer und temperierter Luft zu erzeugen, wobei die staub- und keimarme Luft durch ein Belüftungssystem bereitgestellt wird, das in den Figuren nicht dargestellt ist. Der von den Luftdüsen 21 eingenommene Bereich der Deckenfläche 12 ist beispielsweise kreisförmig, elliptisch, quadratisch oder rechteckig und kann eine Gesamtfläche von mehreren Quadratmetern aufweisen.

Vertikal über dem Behandlungstisch 13 und dem Operationsfeld 15 ist eine Raumkamera 24 unter der Deckenfläche 12, insbesondere unmittelbar unter der Deckenfläche 12 angeordnet. Abweichend von der Darstellung in Figur 1 kann die Raumkamera 24 so in der Decke des medizinischen Behandlungsraums angeordnet sein, dass nur eine Lichteintrittsfläche der Raumkamera 24 auf Höhe der Deckenfläche oder unmittelbar darunter angeordnet ist. Die Raumkamera 24 kann eine Störung des von den Luftdüsen 21 erzeugten Strömungsfelds 22 darstellen und ist deshalb insbesondere möglichst klein und/oder möglichst strömungsgünstig ausgebildet.

Die Raumkamera 24 ist zur Erfassung des Operationsfelds 15 oder zur Erfassung eines größeren Raumbereichs, der den gesamten Behandlungstisch 13 und sich am Behandlungstisch 13 aufhaltendes medizinisches Personal 16 umfassen kann. Die Raumkamera 24 kann eine Laufzeitkamera oder eine Stereokamera zur dreidimensionalen Erfassung des Operationsfelds 15 oder eines größeren Bereichs sein. Anstelle einer Raumkamera 24 oder zusätzlich zu dieser können mehrere Kameras mit unterschiedlichen Blickrichtungen und/oder mit unterschiedlichen Funktionen vorgesehen sein, beispielsweise eine Laufzeitkamera und eine zweidimensional hochauflösende Kamera ohne Erfassung einer dritten Dimension.

Der von den Luftdüsen 21 an der Deckenfläche 12 eingenommene Bereich wird von einem, insbesondere kreisringförmigen, Deckenversorgungssystem bzw. einer Komponente 23 eines Deckenversorgungssystems umschlossen. In das Deckenversorgungssystem 23 sind insbesondere eine Allgemeinbeleuchtung 25 zur im Wesentlichen homogenen Ausleuchtung des gesamten medizinischen Behandlungsraums oder eines zentralen Bereichs des medizinischen Behandlungsraums 10 und eine Operationsleuchte 26 zum Beleuchten des Operationsfelds 15 integriert. Die Operationsleuchte 26 umfasst insbesondere eine Vielzahl von Einzelstrahlern, die jeweils schmale Lichtkegel erzeugen, und die hinsichtlich der Richtung der Lichtemission und/oder der Intensität bzw. des erzeugten Lichtflusses und/oder hinsichtlich der Größe der jeweils ausgeleuchteten Raumwinkelbereiche und/oder hinsichtlich der Richtung der Lichtemission einzeln oder in Gruppen einstellbar bzw. steuerbar sein können. Ferner sind in das Deckenversorgungssystem 23 Schienen 31, 33 integriert, die jeweils ebenfalls kreisringförmig den von den Luftdüsen 21 eingenommenen Bereich der Deckenfläche 12 umschließen. Abweichend von der Darstellung in Figur 1 kann das Deckenversorgungssystem 23 teilweise oder vollständig in die Decke des medizinischen Behandlungsraums 10 integriert sein, so dass die Unterseite des Deckenversorgungssystems 23 mit der Deckenfläche 12 bündig abschließt.

Mehrere Einrichtungen, Vorrichtungen oder Systeme sind jeweils an einer der Schienen 31, 33 befestigt bzw. hängen an jeweils einer der Schienen 31, 33 und sind entlang den durch die Schienen 31, 33 vorgegebenen Pfaden parallel zur Deckenfläche 12 manuell oder motorisch bewegbar bzw. verschiebbar. Einige Beispiele sind nachfolgend mit Bezug auf Figur 1 und die weiteren Figuren dargestellt.

Eine Laufkatze 43 ist an der ersten Schiene 31 des Deckenversorgungssystems 23 angeordnet und trägt über einen Träger 46 eine Projektionsfläche 41. An der Laufkatze 43 ist eine Antriebseinrichtung 44 zum elektromotorischen Bewegen der Laufkatze 43 und mit ihr des Trägers 46 und der Projektionsfläche 41 entlang des durch die erste Schiene 31 vorgegebenen Pfads vorgesehen. Die Projektionsfläche 41 weist insbesondere die Gestalt eines Ausschnitts einer Kugeloberfläche oder einer Oberfläche eines zwei- oder dreiachsigen Ellipsoids auf. Der Rand 410 der Projektionsfläche 41 liegt beispielsweise in einer Ebene und ist kreisförmig oder im Wesentlichen kreisförmig oder weist die Gestalt einer Schnittlinie eines Zylindermantels mit einer Kugeloberfläche auf, wobei der Zylinder insbesondere einen im Wesentlichen rechteckigen Querschnitt mit abgerundeten Ecken aufweist.

Die Projektionsfläche 41 wird durch eine dem medizinischen Personal 16 zugewandte Oberfläche eines entsprechenden, beispielsweise kugelschalenförmigen Körpers gebildet, auf dessen Strukturmerkmale hier nicht näher eingegangen wird.

Eine weitere Laufkatze 53 ist entlang des durch die zweite Schiene 33 vorgegebenen Pfads bewegbar und trägt einen Projektor bzw. ein Projektionssystem 50 zum Projizieren eines bewegten oder unbewegten Bilds, einer Grafik, von Text, Zahlen, Symbolen und anderen Darstellungen von Information auf die Projektionsfläche 41. Der Projektor 50 weist hinsichtlich der Projektion von Licht insbesondere Eigenschaften und Merkmale auf, die herkömmlich einem Videobeamer zugeschrieben werden. Eine Antriebseinrichtung 54 an der weiteren Laufkatze 53 ermöglicht eine motorische Bewegung der weiteren Laufkatze 53 und des Projektors 50 entlang des durch die zweite Schiene 33 vorgegebenen Pfads.

Der Projektor 50 und die Projektionsfläche 41 bilden zusammen eine Anzeigevorrichtung zum Anzeigen von medizinisch relevanter Information für medizinisches Personal 16. Neben den Antriebseinrichtungen 44, 54 an den Laufkatzen 43, 53 können weitere Antriebseinrichtungen an der Projektionsfläche 41 und/oder am Projektor 50 vorgesehen sein, um diese jeweils in eine oder mehrere Richtungen, insbesondere in horizontaler und vertikaler Richtung schwenken zu können. Dadurch können die Projektionsfläche jederzeit auf das medizinische Personal 16 und/oder auf den Projektor 50 und der Projektor 50 jederzeit auf die Projektionsfläche 41 ausgerichtet werden.

Eine weitere Laufkatze 63 an der ersten Schiene 31 trägt eine Tragvorrichtung 60 für ein oder mehrere medizinische Geräte 69. Mittels einer Antriebseinrichtung 64 an der weiteren Laufkatze 63 sind die weitere Laufkatze 63 und mit ihr die Tragvorrichtung 60 entlang des durch die erste Schiene 31 vorgegebenen Pfads bewegbar. Die Tragvorrichtung 60 umfasst einen Gerätehalter 67 und einen vertikalen Holm 66, der den Gerätehalter 67 mit der Laufkatze 63 verbindet und im oberen Bereich gekröpft ausgebildet sein kann, wie dies in Figur 1 dargestellt ist. Der vertikale Holm 66 ist bei dem in Figur 1 dargestellten Beispiel teleskopierbar und bildet somit eine vertikale Führungseinrichtung für den Gerätehalter 67 und von dem Gerätehalter 67 gehaltene medizinische Geräte 69, die eine durch einen Doppelpfeil angedeutete vertikale Bewegung des Gerätehalters 67 und der medizinischen Geräte 69 ermöglicht.

Der Gerätehalter 67 ist bei dem in Figur 1 dargestellten Beispiel als im Wesentlichen horizontale Tragplatte ausgebildet, auf dem das oder die medizinischen Geräte 69 stehen. Eine oder mehrere in Figur 1 nicht dargestellte Antriebseinrichtungen können zur Rotation des Gerätehalters 67 oder der gesamten Tragvorrichtung 60 um eine vertikale Achse und/oder zum Bewegen des Gerätehalters 67 in vertikaler Richtung vorgesehen und ausgebildet sein.

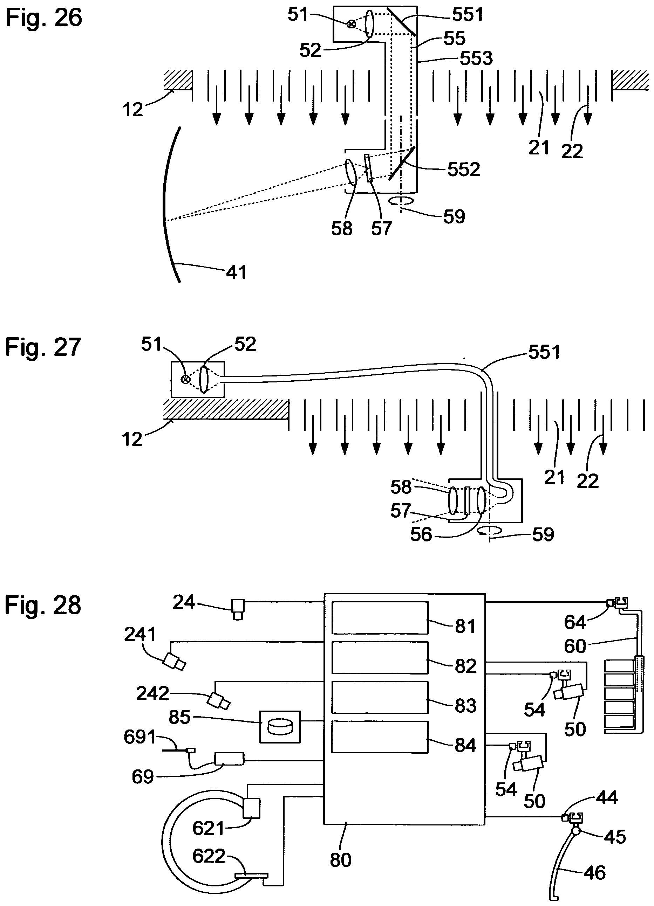

Zusätzlich zu den in Figur 1 gezeigten oder nicht gezeigten und oben beschriebenen Antriebseinrichtungen 43, 53, 63 oder teilweise oder vollständig in diese integriert können Positionssensoren vorgesehen sein, um die Positionen der Laufkatzen 43, 53, 63 entlang der durch die Schienen 31, 33 vorgegebenen Pfade, Winkelpositionen und Positionen in vertikaler Richtung der Projektionsfläche 41, des Projektors 50 und/oder des Gerätehalters 67 zu erfassen. Diese Sensoren können mit einer unten anhand der Figur 28 dargestellten Steuerung gekoppelt sein. Soweit Schrittmotoren in den Antriebseinrichtungen verwendet werden, können die entsprechenden Positionen bzw. Koordinaten alternativ durch Zählen der zurückgelegten Schritte bestimmt werden.

Die unten anhand der Figur 28 dargestellte Steuerung kann die Antriebseinrichtungen in Abhängigkeit von den erfassen Positionen und optional ferner in Abhängigkeit von der Position oder den Positionen relevanten medizinischen Personals oder aufgrund von mittels der Raumkamera 24 oder anderer Sensoren erfassten Gesten des medizinischen Personals steuern. Die Steuerung kann somit jederzeit eine für medizinisches Personal unter Gesichtspunkten der Ergonomie optimale Anordnung und Ausrichtung der Projektionsfläche 41 und der durch die Tragvorrichtung 60 getragenen medizinischen Geräte 69 bewirken. Der weitgehende oder - wie in Figur 1 angedeutet - vollständige Verzicht auf herkömmliche Deckenarme kann ein störungsarmes oder störungsfreies vertikales laminares Strömungsfeld 22 ermöglichen.

Figur 2 zeigt eine weitere schematische Darstellung des medizinischen Behandlungsraums aus Figur 1. Die Zeichenebene der Figur 2 ist senkrecht zur Zeichenebene der Figur 1 und parallel zur Bodenfläche 11 und zur Deckenfläche 12 des medizinischen Behandlungsraums 10. Die Schienen 31, 33 und die durch die Schienen 31, 33 vorgegebenen Pfade 37, 39 umschließen kreisförmig den Bereich der Deckenfläche, der über dem Behandlungstisch 13 angeordnet ist. Die Laufkatze 43 und mit ihr die Projektionsfläche 41, die Laufkatze 53 und mit ihr der Projektor 50 und die Laufkatze 63 und mit ihr die Tragvorrichtung 60 sind entlang den durch die Schienen 31, 33 vorgegebenen Pfaden 37, 39 bewegbar. Die Projektionsfläche 41 und die Tragvorrichtung 60 sind in Figur 2 in der gleichen Position dargestellt wie in Figur 1, der Projektor 50 ist in einer Position gezeigt, die gegenüber der in Figur 1 gezeigten Position leicht verschoben ist.

In den Figuren 1 und 2 sind in gestrichelten Linien die Konturen der Kugeloberfläche angedeutet, auf der die Projektionsfläche 41 liegt. Bei der in den Figuren 1 und 2 dargestellten Situation befinden sich der Kopf 17 bzw. die Augen des medizinischen Personals 16 näherungsweise auf dieser Kugeloberfläche 48. Diese Konfiguration hat sich beispielsweise bei Betrachtung von Bildern, die durch ein Endoskop mit einem Bildwinkel von 70 Grad erfasst werden, als näherungsweise optimal hinsichtlich der Erzielung eines immersiven und dreidimensionalen Bildeindrucks bei dem medizinischen Personal 16 herausgestellt.

Im Unterschied zur Figur 1 sind in Figur 2 ferner weitere, nicht mit Bezugszeichen versehene Projektoren gezeigt, die abwechselnd oder gleichzeitig betrieben werden können, um eine gleichmäßige Ausleuchtung der Projektionsfläche 41 zu ermöglichen.

Figur 3 zeigt eine schematische Darstellung eines weiteren medizinischen Behandlungsraums, der dem oben anhand der Figuren 1 und 2 dargestellten medizinischen Behandlungsraums in einigen Merkmalen ähnelt. Im Unterschied zu dem Beispiel der Figuren 1 und 2 weisen beim Beispiel der Figur 3 die Schienen 31, 33 und die durch die Schienen 31, 33 vorgegebenen Pfade 37, 39 jeweils eine elliptische Gestalt auf. Eine elliptische oder ovale Gestalt der Pfade 37, 39 kann beispielsweise bei beengten räumlichen Verhältnissen vorteilhaft sein. Bei der in Figur 3 gezeigten Konfiguration sind sowohl die Projektionseinrichtungen 50 als auch der Projektionsschirm 41 und die Tragvorrichtung 60 jeweils gegenüber den zugeordneten Laufkatzen 53, 43 bzw. 63 gedreht.

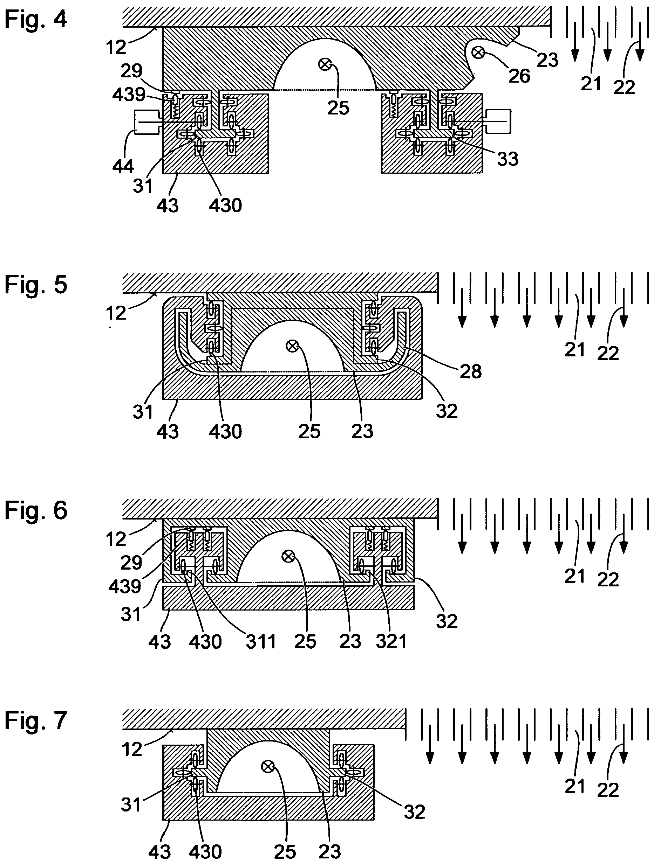

Die Figuren 4 bis 9 zeigen schematische Darstellungen verschiedener Ausführungsformen von Deckenversorgungssystemen 23 und insbesondere der durch Schienen 31, 32, 33, 34 an den Deckenversorgungssystemen 23 gebildeten Schieneneinrichtungen, sowie von Laufkatzen 43, 53 an den Schienen 31, 32, 33, 34. Die Darstellungen haben jeweils weitgehend den Charakter von Schnittdarstellungen, wobei die Zeichenebenen bzw. die dargestellten Schnittebenen der Zeichenebene der Figur 1 entsprechen und somit im medizinischen Behandlungsraum vertikal angeordnet sind.

In den Figuren 4 bis 9 ist jeweils eine Raumbeleuchtung 25 mit einer Lichtquelle und einem angedeuteten Reflektor dargestellt. Strukturelle Details der Deckenversorgungssysteme 23 und der Laufkatzen 43, 53 sind jeweils allenfalls angedeutet. Auch die Decke des medizinischen Behandlungsraums ist in den Figuren 4 bis 9 nur jeweils durch die Deckenfläche 12 und die Luftdüsen 21 zum Erzeugen des vertikalen laminaren Strömungsfelds 22 angedeutet.

Einige der anhand der Figuren 4 bis 9 dargestellten Ausführungsformen können alternativ insofern in die Decke des medizinischen Behandlungsraums integriert sein, als die Unterkante des Versorgungssystems 23 oder die Unterkanten der Schienen 31, 32, 33, 34 mit der Deckenfläche bündig angeordnet sind.

Bei dem in Figur 4 dargestellten Beispiel sind Schienen 31, 33 vorgesehen, an denen jeweils eine Laufkatze 43 angeordnet ist. Die Schienen 31, 33 können parallel oder im Wesentlichen parallel zueinander angeordnet sein, bzw. einen konstanten Abstand aufweisen. Alternativ können die Schienen 31, 33 einen variierenden Abstand aufweisen. Jede der beiden Schienen 31, 33 weist einen Querschnitt auf, der im Wesentlichen einen um ein 180 Grad gedrehten "T" entspricht. Jede Laufkatze 43 weist mehrere Laufrollen 430 auf, deren Achsen jeweils senkrecht oder im Wesentlichen senkrecht zu der lokalen Richtung des durch die Schiene 31 bzw. 33 vorgegebenen Pfads 37 bzw. 39 (vgl. Figuren 2, 3) sind. Jeweils eine oder mehrere Laufrollen 430 sind über einer nach oben gerichteten horizontalen Lauffläche an der Schiene 31, 33 angeordnet, um die Gewichtskraft der Laufkatze 43 und daran befestigter Einrichtungen auf die Schiene 31, 33 zu übertragen. Jeweils eine oder mehrere Laufrollen 430 liegen an seitlichen bzw. vertikalen Laufflächen oder nach unten gerichteten horizontalen Laufflächen der Schienen 31, 33 an, um horizontale Kräfte, vertikal nach oben gerichtete Kräfte und Kippmomente auf die Schienen 31, 33 abzuleiten. Jeweils ein oder mehrere Laufräder 430 können durch eine Antriebseinrichtung 44 angetrieben werden, um die Laufkatze 43 entlang der entsprechenden Schiene 31, 33 zu bewegen bzw. zu verschieben.

Ferner ist parallel zu jeder Schiene 31, 33 eine Kontaktschiene 29 aus einem elektrisch leitfähigen Material vorgesehen. An der Laufkatze 43 ist ein Schleifkontakt 439 so angeordnet, dass er an der Kontaktschiene 29 anliegt und eine elektrisch leitfähige Verbindung mit dieser bildet. Eine Spiralfeder oder ein anderes elastisches Element kann vorgesehen sein, um den Schleifkontakt 439 gegen die Kontaktschiene 29 zu drücken. Der Schleifkontakt 439 bildet somit eine Kontakteinrichtung zur Bildung eines elektrisch leitfähigen Kontakts mit der Kontaktschiene 29. Die Kontaktschiene 29 und der Schleifkontakt 439 können zur Übertragung von elektrischer Leistung und von elektrischen Signalen zwischen dem Deckenversorgungssystem 23 und der Laufkatze 43 vorgesehen sein. Der zum Schließen eines Stromkreises erforderliche zweite Pol wird beispielsweise durch die Schiene 31, 33 gebildet. Alternativ oder zusätzlich können weitere Kontaktschienen vorgesehen sein, an denen weitere Schleifkontakte anliegen.

Neben elektrischer Leistung können über die Kontaktschienen 29 und die Schleifkontakte 439 insbesondere niederfrequente Signale bzw. Signale mit niedrigen Datenraten oder Bandbreiten übertragen werden. Insbesondere werden mittels der Kontaktschienen 29 und der Schleifkontakte 439 Steuersignale für die Antriebseinrichtung 44 und in umgekehrter Richtung Positionssignale, die Positionen in den entsprechenden Freiheitsgraden anzeigende, übertragen. Zumindest wenn die Laufkatze 43 nicht bewegt wird, können ferner Signale mit höheren Datenraten bzw. Bandbreiten übertragen werden, beispielsweise Bildsignale.

Figur 5 zeigt eine schematische Darstellung einer Ausführungsform, die in einigen Merkmalen der Ausführungsform der Figur 4 ähnelt. Bei der Ausführungsform der Figur 5 greift die Laufkatze 43 in zwei gegenüberliegende, voneinander abgewandte U-förmige Schienen 31, 32 ein und ist darin jeweils mit mehreren Laufrollen 430 geführt. Die Laufkatze 43 umgreift dabei ferner Blenden 28. Die Blenden 28 verdecken die Schienen 31, 33 teilweise oder vollständig, so dass diese für einen Betrachter im medizinischen Behandlungsraum nicht sichtbar sind. Die Blenden 28 können ferner die Schienen 31, 32 und die Laufrollen 430 an der Laufkatze 43 vor Verschmutzung und Beschädigung schützen und an den Schienen 31, 32 und an den Laufrollen 430 auftretenden Abrieb aufnehmen bzw. zurückhalten.

Im Unterscheid zu den Figuren 4 und 6 bis 9 sind in Figur 5 ferner Beispiele einfacher struktureller Merkmale des Deckenversorgungssystems angedeutet. Die Schienen 31, 32 sind Bestandteile eines Profilbauteils, das zunächst an der Deckenfläche 12 verankert werden kann. Danach können die Raumbeleuchtung 25 und die Blenden 28 insbesondere als Einheit in das Profilbauteil eingesetzt werden.

Kontaktschienen und Schleifkontakte wie bei dem Beispiel der Figur 4 sind in Figur 5 nicht gezeigt, können jedoch auch bei der Ausführungsform der Figur 5 vorgesehen sein.

Figur 6 zeigt eine schematische Darstellung einer weiteren Ausführungsform, bei der die Laufkatze 43 durch zwei Schienen 31, 32 geführt und gehalten ist. Jede Schiene 31, 32 weist einen nach unten offenen C-förmigen Querschnitt auf. Laufrollen 430 der Laufkatze 43 sind innerhalb der C-förmigen Querschnitte der Schienen 31, 32 angeordnet und stützen sich dort auf horizontale oder im Wesentlichen horizontale Laufflächen ab. Zur Führung der Laufkatze 43 in horizontaler Richtung quer zu den durch die Schienen 31, 32 vorgegebenen Pfaden sind beispielsweise die in Figur 6 angedeuteten, an den jeweils einander gegenüberliegenden Rändern einer Schiene 31. 32 nach oben ragenden Führungsstege 311, 321 für die Laufrollen 430 vorgesehen. Alternativ oder zusätzlich können weitere Laufrollen vorgesehen sein, die an vertikalen Laufflächen in den Schienen 31, 32 anliegen, um horizontale Kräfte auf die Schienen 31, 32 zu übertragen.

In den nach unten offenen Hohlräumen in den Schienen 31, 32 sind jeweils eine oder - wie in Figur 6 angedeutet - mehrere Kontaktschienen 29 angeordnet. An der Laufkatze 43 sind korrespondierende Schleifkontakte 439 vorgesehen.

Figur 7 zeigt eine schematische Darstellung einer weiteren Ausführungsform, bei der zwei parallele Schienen 31, 32 an voneinander abgewandten Seiten des Deckenversorgungssystems 23 angeordnet sind. Die Schienen 31, 32 stehen jeweils stegförmig horizontal vor. Die Laufkatze 43 weist Laufrollen 430 auf, die an nach oben gerichteten horizontalen, an vertikalen bzw. an nach unten gerichteten horizontalen Laufflächen anliegen und eine Ableitung bzw. Übertragung von Kräften und Momenten auf die Schienen 31, 32 ermöglichen.

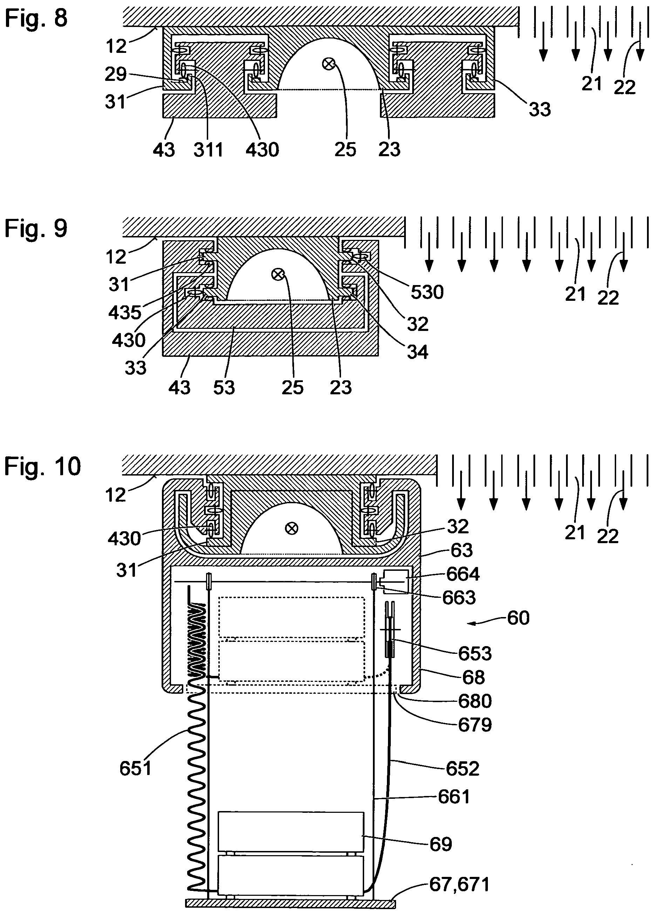

Figur 8 zeigt eine schematische Darstellung einer Ausführungsform, bei der ähnlich wie bei der Ausführungsform der Figur 4 zwei Laufkatzen 43 unabhängig voneinander von jeweils einer Schiene 31, 33 geführt und gehalten sind. Die einzelnen Schienen 31, 33 und die Führung der Laufkatzen 43 in den Schienen 31, 33 ähneln der Ausführungsform der Figur 6. Im Unterschied zur Ausführungsform der Figur 6 sind bei der Ausführungsform der Figur 8 alternativ zu Führungsstegen 311 oder - wie in Figur 8 angedeutet - zusätzlich zu diesen Laufrollen 430 vorgesehen, die an vertikalen Laufflächen in den Schienen 31, 33 anliegen. Ferner sind Kontaktschienen 29 in den Schienen 31, 33 vorgesehen, die Laufflächen für jeweils ein oder mehrere Laufräder 430 bilden. Eine Übertragung von elektrischer Leistung und/oder elektrischen Signalen zu oder von den Laufkatzeneinrichtungen 43 kann über an den Kontaktschienen 29 anliegende Laufrollen 430 erfolgen.

Figur 9 zeigt eine schematische Darstellung einer weiteren Ausführungsform, bei der ähnlich wie bei der Ausführungsform der Figur 7 an voneinander abgewandten Seiten des Deckenversorgungssystems 23 horizontal vorstehende stegförmige Schienen 31, 32, 33, 34 vorgesehen sind. Im Unterschied zu der Ausführungsform der Figur 7 sind zwei Paare von Schienen 31, 32 bzw. 33, 34 vorgesehen. Eine erste Laufkatze 43 ist von einem ersten Paar von Schienen 31, 32 gehalten und geführt. Eine zweite Laufkatze 53 ist von einem zweiten Paar von Schienen 33, 34 gehalten und geführt. Durch eine entsprechende Ausgestaltung der Laufkatzen 43, 53, wie sie beispielhaft in Figur 9 angedeutet ist, können die Laufkatzen 43 ,53 unabhängig voneinander entlang der durch die Schienen 31, 32 bzw. 33, 34 vorgegebenen Pfade bewegt werden, ohne miteinander zu kollidieren.

Im Unterschied zu den Ausführungsformen der Figuren 4 bis 8 sind bei der Ausführungsform der Figur 9 anstelle von Laufrollen 430 überwiegend Gleitbeläge 435 vorgesehen. Zusätzlich zu den Gleitbelägen ist jeweils eine Laufrolle 430, 530 vorgesehen, die durch eine in Figur 9 nicht dargestellte Antriebseinrichtung angetrieben sein kann, um die zugeordnete Laufkatze 43, 53 zu bewegen.

Bei den Ausführungsformen der Figuren 4 bis 8 können die Laufrollen 430 teilweise oder vollständig durch Gleitbeläge 435 ersetzt werden, wie sie oben anhand der Figur 9 beschrieben sind. Bei der Ausführungsform der Figur 9 können die Gleitbeläge 435 teilweise oder vollständig durch Laufrollen ersetzt werden. Insbesondere können bei jeder der anhand der Figuren 4 bis 9 dargestellten Ausführungsformen Laufrollen zur Aufnahme von Gewichtkräften und Gleitbeläge zur horizontalen Führung und zur Aufnahme von anderen Kräften und Momenten vorgesehen sein. Es kann vorteilhaft sein, jeweils ein angetriebenes Laufrad zum Bewegen der Laufkatze vorzusehen. Um einen Antrieb ohne Schlupf zu ermöglichen, können ein angetriebenes Laufrad als Zahnrad und eine gegenüberliegende Lauffläche als Zahnstange ausgebildet sein. Alternativ zu einem form- oder kraftschlüssig mit der zugeordneten Lauffläche wechselwirkenden Laufrolle sind ein elektromagnetischer Linearantrieb, ein Ultraschallmotor, ein Antrieb über einen Seilzug oder eine manuelle Bewegbarkeit möglich.

Die Laufrollen oder Gleitbeläge der Ausführungsformen der Figuren 4 bis 9 können teilweise oder vollständig durch Einrichtungen zum magnetischen Anheben und Führen der Laufkatzen 43 ersetzt werden. Dazu sind insbesondere Permanent- und/oder Elektromagneten und Einrichtungen zur Regelung konstanter Abstände vorgesehen. Die Einrichtungen zum magnetischen Anheben und Führen der Laufkatzen 43 können ferner ausgebildet sein, um die Laufkatzen magnetisch anzutreiben.

In den Figuren 4 bis 9 wurde für die Laufkatzen beispielhaft das Bezugszeichen 43 verwendet, bei Figur 9 auch das Bezugszeichen 53. Die Bezugszeichen 43, 53 beziehen sich bei den Darstellungen in den Figuren 1 bis 3 auf die Laufkatzen, die den Projektionsflächen 41 bzw. den Projektoren 50 zugeordnet sind. Die in den Figuren 4 bis 9 gezeigten Laufkatzen können jedoch auch der Tragvorrichtung 60 aus den Figuren 1 bis 3 zugeordnet sein oder andere Einrichtungen tragen, insbesondere auch die nachfolgend anhand der Figuren 10 bis 24 dargestellten Einrichtungen.

Figur 10 zeigt eine schematische Darstellung einer weiteren Tragvorrichtung 60 mit einer zu der in den Figuren 1 bis 3 alternativen Ausgestaltung. Die Darstellung in Figur 10 weist teilweise den Charakter einer Schnittdarstellung auf, wobei die Zeichenebene den Zeichenebenen der Figuren 1 und 4 bis 9 entspricht.

Die Laufkatze 63 ist mit einem Behälter 68 verbunden oder - wie in Figur 10 angedeutet - einstückig ausgebildet. An der Unterseite des Behälters 68 ist eine Öffnung 680 vorgesehen. Der Gerätehalter 67 ist bei dieser Ausführungsform nicht an einem vertikalen teleskopierbaren Holm geführt, sondern an mehreren Seilen 661 aufgehängt, deren oberen Enden auf Haspeln 663 aufgewickelt werden können. Die Haspeln 663 können durch einen Elektromotor 664 angetrieben werden, um die Seile 661 auf- oder abzuwickeln und damit den Gerätehalter 67 vertikal nach oben bzw. nach unten zu bewegen. Somit bilden die Seile 661, die Haspeln 662 und der Elektromotor 664 eine Hubeinrichtung für den Gerätehalter 67.

In Figur 10 ist der Gerätehalter 67 zusammen mit zwei medizinischen Geräten 69 in einer unteren Position bzw. Betriebsposition 671 in durchgezogenen Linien dargestellt. Der Gerätehalter 67 ist ferner zusammen mit den medizinischen Geräten 69 in gestrichelten Linien in einer oberen Ruheposition 679 dargestellt. In der Ruheposition 679 verschließt der Gerätehalter 67 die Öffnung 680 an der Unterseite des Behälters 68. Die Geräte 69 sind in dem durch den Behälter 68 gebildeten Hohlraum angeordnet und dort gegen Verschmutzung und Beschädigung weitgehend geschützt. Durch eine optisch ruhige Gestaltung der Außenseite des Behälters 68 und der Unterseite des Gerätehalters 67 entsteht ein ruhiger optischer Eindruck, der nicht geeignet ist, medizinisches Personal von seinen Aufgabe abzulenken und die Konzentration auf relevante Informationsquellen unterstützen kann.

Viele medizinische Geräte 69 sind auf eine Versorgung mit elektrischer Leistung, Beleuchtungslicht von einer Hochleistungslichtquelle, Druckluft oder anderen Fluiden sowie auf einen Austausch elektrischer oder optischer Signale (beispielsweise über einen Storz Communication Bus SCB) mit anderen Geräten angewiesen. Um eine Inbetriebnahme und Außerbetriebnahme der Geräte 69 ohne vorherige Herstellung oder Trennung von Leitungsverbindungen zu ermöglichen, können die medizinischen Geräte 69 ständig mit Leistungs-, Signal-, Licht- und Fluid-Quellen verbunden bleiben, sowohl in der Betriebsposition 671 als auch in der Ruheposition 679 des Gerätehalters 67 und in allen Positionen dazwischen.

Zu diesem Zweck sind alternativ oder gleichzeitig eine oder mehrere dehnbare Leitungen 651 und/oder eine oder mehrere aufrollbare Leitungen 652 zwischen dem Inneren des Behälters 68 und dem Gerätehalter 67 oder den auf dem Gerätehalter 67 angeordneten medizinischen Geräten 69 vorgesehen. Die dehnbare Leitung 651 weist insbesondere eine helikale Gestalt auf, ähnlich einem allgemein als Spiralkabel bezeichneten Kabel, wie es herkömmlichen zwischen einem Telefonapparat und dessen Hörer vorgesehen ist. Die aufrollbare Leitung 652 ist auf eine Haspel 653 aufrollbar. Die Haspel 653 kann motorisch oder durch eine Feder angetrieben sein, um die aufrollbare Leitung 652 selbsttätig aufzurollen, wenn der Gerätehalter 67 mit den medizinischen Geräten 69 von der Betriebsposition 671 in die Ruheposition 679 angehoben wird.

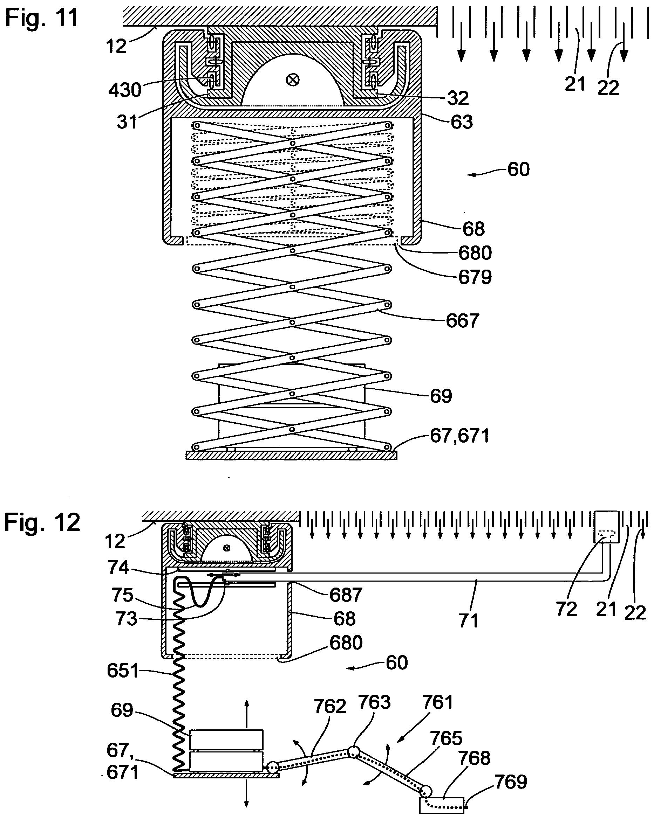

Figur 11 zeigt eine schematische Darstellung einer zu der Ausführungsform der Figur 10 alternativen Ausführungsform einer Tragvorrichtung 60. Die in Figur 11 gezeigte Ausführungsform ähnelt der oben anhand der Figur 10 dargestellten Ausführungsform in einigen Merkmalen. Die Ausführungsform der Figur 11 unterscheidet sich von der Ausführungsform der Figur 10 insbesondere dadurch, dass anstelle mehrerer Seile 661 eine Scherenmechanik 667 vorgesehen ist. Die Scherenmechanik 667 ermöglicht im Gegensatz zu den Seilen der Ausführungsform der Figur 10 eine - von der ihr eigenen Rest-Elastizität abgesehen - starre Führung des Gerätehalters 67. Der Gerätehalter 67 wird durch die Scherenmechanik 667 nicht nur - wie auch durch die Seile der Ausführungsform der Figur 10 - jederzeit in der Horizontalen gehalten. Die Scherenmechanik 667 kann ferner auf den Gerätehalter 67 ausgeübte horizontale Kräfte auf die Laufkatze 63 übertragen. Damit weist die Scherenmechanik 667 die Funktion einer Führungseinrichtung zur weitgehend starren Führung entlang eines vorbestimmten geraden vertikalen Wegs auf.

Ferner kann die Scherenmechanik 667 zum Bewegen des Gerätehalters 67 verwendet werden. Eine beispielsweise lediglich am obersten Paar von Holmen der Scherenmechanik 667 angreifende Kraft hat eine ebenmäßige Verformung der gesamten Scherenmechanik 667 zur Folge. Insofern hat die Scherenmechanik 667 auch die Funktion einer Hubeinrichtung für den Gerätehalter 67.

Alternativ können die Seile der Ausführungsform der Figur 10 mit der Scherenmechanik 667 der Ausführungsform der Figur 11 kombiniert werden. In diesem Fall wirken die Scherenmechanik 667 in erster Linie als weitgehend starre Führung für den Gerätehalter 67, die horizontale Kräfte aufnehmen und auf die Laufkatze 63 übertragen kann, und die Seile als Hubeinrichtungen zum Heben (und Senken) des Gerätehalters 67.

Im Gegensatz zur Figur 10 sind in Figur 11 keine Leitungen zum Übertragen von elektrischer Leistung, elektrischen oder optischen Signalen, Beleuchtungslicht oder Fluiden zwischen dem Behälter 68 und dem Gerätehalter 67 bzw. medizinischen Geräten 69 auf dem Gerätehalter 67 dargestellt. Es können Leitungen vorgesehen sein, wie sie oben anhand der Figur 10 dargestellt wurden. Alternativ können Leitungen entlang den Holmen der Scherenmechanik 667 verlaufen bzw. an diesen (insbesondere punktuell) befestigt sein.

Figur 12 zeigt eine schematische Darstellung einer Tragvorrichtung 60, wie sie oben anhand der Figuren 10 und 11 dargestellt wurde, mit einem Versorgungsarm 71. Der Gerätehalter 67 kann entsprechend einer der oben anhand der Figuren 10 und 11 dargestellten Ausführungsformen oder auf andere Weise geführt und gehoben werden. Entsprechende Merkmale sind deshalb in Figur 12 nicht dargestellt.

Der Versorgungsarm 71 ist an seinem radial inneren Ende über ein Gelenk 72 mit der Decke des medizinischen Behandlungsraums verbunden, wobei das Gelenk 72 ein Schwenken des Versorgungsarms 71 um eine vertikale Achse ermöglicht. Das radial äußere Ende 73 des Versorgungsarms 71 ragt durch eine seitliche Öffnung 687 in den Behälter 68 und ist dort in einer oder mehreren Führungsschienen 74 geführt. Die Führungsschienen 74 ermöglichen insbesondere bei einer nicht-kreisförmigen Gestalt des durch die Schienen der Deckenversorgungseinrichtung vorgegebenen Pfads einen Längenausgleich bzw. eine Kompensation einer Variation des Abstands zwischen dem Gelenk 72 und dem Behälter 68.

Das radial äußere Ende 73 des Versorgungsarms 71 ist insbesondere mittels einer elastischen Leitung 75 mit dem oberen Ende der oben anhand der Figur 10 dargestellten helikalen, dehnbaren Leitung 651 verbunden. Insbesondere sind die dehnbare Leitung 651 und die elastische Leitung 75 durch unterschiedliche Bereiche der gleichen einstückig ausgeführten Leitung gebildet. Die elastische Leitung 75 ermöglicht insbesondere aufgrund einer losen Schlaufe, wie sie in Figur 12 angedeutet ist, mit variabler Geometrie eine Verschiebung des radial äußeren Endes 73 des Versorgungsarms 71 im Behälter 68.

Der Versorgungsarm 71, die elastische Leitung 75 und die dehnbare Leitung 651 sind insbesondere zum Leiten eines oder mehrerer verschiedener Fluide zu oder von einem der Geräte 69 auf dem Gerätehalter 67 ausgebildet. Dazu weist das Gelenk 72 insbesondere eine Abdichtung mittels einer oder mehrerer Radial-Wellendichtungen (oft auch als Simmerringe bezeichnet) oder anderer Dichtungen auf. Alternativ oder zusätzlich sind der Versorgungsarm 71, die elastische Leitung 75 und die dehnbare Leitung 651 zum Leiten elektrischer Leistung, elektrischer oder optischer Signale oder von Beleuchtungslicht ausgebildet. Dazu können am oder - in der Regel vorteilhaft - im Versorgungsarm jeweils eine oder mehrere entsprechende Leitungen angeordnet sein.

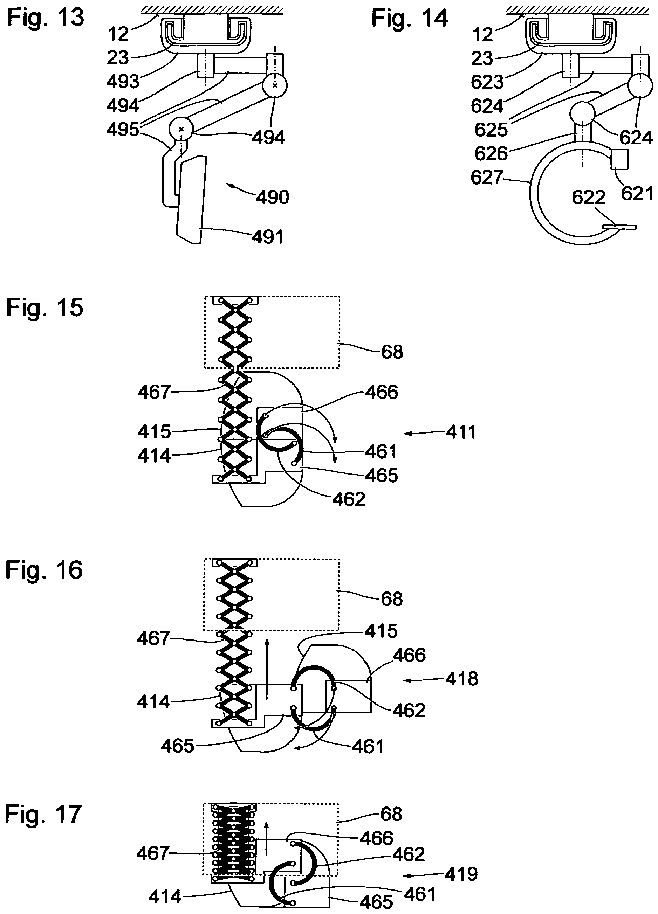

Der Versorgungsarm ist nicht ausgebildet, um Kräfte aufzunehmen, die über sein Eigengewicht hinausgehen. Der Querschnitt des Versorgungsarms 71 kann deshalb klein sein und für eine minimale Störung des vertikalen laminaren Strömungsfelds 22 ausgebildet sein, beispielsweise durch eine Tropfenform. Ein Versorgungsarm mit den beschriebenen Eigenschaften ist auch mit anderen Tragvorrichtungen als den anhand der Figuren 10 bis 12 dargestellten verwendbar, beispielsweise mit der oben anhand der Figuren 1 bis 3 Dargestellten. Ferner ist ein Versorgungsarm 71 mit den hier dargestellten Eigenschaften zur Zuführung elektrischer Leistung und elektrischer oder optischer Signale zu einem Projektor, zu einer Projektionsfläche oder zu einer anderen Anzeigevorrichtung oder zu einer der unten anhand der Figuren 13 und 14 dargestellten Vorrichtungen verwendbar.

In Figur 12 ist ferner ein Bereitstellungsarm 761 mit mehren Holmen 762, die mit Gelenken 763 untereinander und mit dem Gerätehalter 67 gelenkig verbunden sind, dargestellt. Der Bereitstellungsarm 761 trägt an seinem vom Gerätehalter 67 beabstandeten Ende eine Satelliteneinrichtung 768. An oder in dem Bereitstellungsarm 761 können eine oder mehrere Leitungen 765 zum Übertragen von elektrischer Leistung, elektrischen oder optischen Signalen, Beleuchtungslicht (beispielsweise für Endoskopie) und Fluiden angeordnet sein. Die Leitung oder die Leitungen 765 koppeln ein oder mehrere Geräte 69 auf dem Gerätehalter 67 mit der Satelliteneinrichtung 768. Die Satelliteneinrichtung 768 kann Bedienelemente bzw. eine Benutzerschnittstelle bereitstellen, um Funktionen von einem oder mehreren medizinischen Geräten 69 auf dem Gerätehalter 67 zu steuern oder zu überwachen. Ferner kann die Satelliteneinrichtung 768 einen oder mehrere Anschlüsse 769 aufweisen, an denen beispielsweise von einem der medizinischen Geräte 69 erzeugtes Beleuchtungslicht bereitgestellt wird.

Aufgrund der Ausbildung des Bereitstellungsarms 761 mit einem oder mehreren Gelenken 763 kann die Satelliteneinrichtung 768 von medizinischem Personal in ergonomisch optimaler Weise angeordnet werden.

Abweichend von der Darstellung in Figur 12 kann der Bereitstellungsarm 761 statt mit dem Gerätehalter 67 alternativ mit dem Behälter 68, mit dem Versorgungsarm 71, mit dem Gelenk 72 des Versorgungsarms 71, nahe dem Gelenk 72 des Versorgungsarms 71 mit der Decke des medizinischen Behandlungsraums 10 oder mit einem anderen Befestigungspunkt gelenkig mechanisch verbunden sein. Im Fall einer mechanischen Befestigung des Bereitstellungsarms 71 am Gelenk 72 des Versorgungsarms 71 kann der Bereitstellungsarm 71 gemeinsam mit dem Versorgungsarm 71 oder unabhängig von diesem rotierbar bzw. schwenkbar sein. Im Fall einer mechanischen Befestigung des Bereitstellungsarms 761 am Versorgungsarm 71 ist der Versorgungsarm 71 anders als oben beschrieben auch als Tragarm zur Aufnahme bzw. Übertragung bzw. Ableitung mechanischer Kräfte und Momente ausgebildet.

Der Bereitstellungsarm 761 kann mit dem Gerätehalter 67, mit dem Behälter 68, mit dem Versorgungsarm 71, mit dem Gelenk 72 des Versorgungsarms 71, mit der Decke des medizinischen Behandlungsraums 10 oder mit einem anderen Befestigungspunkt lediglich gelenkig mechanische verbunden sein. Alternativ können einer oder mehrere lineare translatorische Freiheitsgrade vorgesehen sein, die insbesondere eine Translation des von der Satelliteneinrichtung 768 beabstandeten Endes des Bereitstellungsarms 761 in horizontaler und/oder vertikaler Richtung ermöglichen. Beispielsweise sind eine oder mehrere Führungseinrichtungen vorgesehen, die jeweils eine Verschiebung des von der Satelliteneinrichtung 768 beabstandeten Endes des Bereitstellungsarms entlang eines geraden oder gekrümmten Wegs ermöglichen. Insbesondere kann das von der Satelliteneinrichtung 768 beabstandete Ende des Bereitstellungsarms 761 entlang des Versorgungsarms 71 verschiebbar sein.

Figur 13 zeigt eine schematische Darstellung einer Anzeigevorrichtung 490, die sich von der Anzeigevorrichtung, die durch die oben anhand der Figuren 1 bis 3 dargestellten Projektionsfläche 41 und Projektor 50 gebildet wird, unterscheidet. Ein Bildschirm 491 ist über mehrere Gelenke 494 und Holme 495 mit einer Laufkatze 493 mechanisch verbunden. Die Laufkatze kann Merkmale der oben anhand der Figuren 4 bis 9 dargestellten Laufkatzen aufweisen und ermöglicht eine Bewegung entlang eines Deckenversorgungssystems 23 an einer Deckenfläche 12. Bei dem dargestellten Beispiel ist ein Holm 495 lediglich in einer horizontalen Ebene bzw. um eine vertikale Achse schwenkbar, ein weiterer Holm 495 ist um eine vertikale Achse und um eine horizontale Achse schwenkbar.

Figur 14 zeigt eine schematische Darstellung einer Tragvorrichtung zum Tragen eine Röntgenquelle 621 und eines ortsempfindlichen Röntgendetektors 622 bzw. eines für Röntgenstrahlung empfindlichen Bildsensors. Die Röntgenquelle 621 und der Röntgendetektor 622 sind über einen C-förmigen Bogen 627 mechanisch starr miteinander verbunden. Der C-förmige Bogen 627 ist über mehrere Gelenke 624 und mehrere Holme 625, 626 mit einer Laufkatze 623 verbunden. Die Laufkatze 623 weist insbesondere Merkmale auf, die oben anhand der Figuren 4 bis 9 beschrieben sind. Die Laufkatze 623 ermöglicht eine Bewegung entlang des durch Schienen am Deckenversorgungssystem 23 vorgegebenen Pfads parallel zur Deckenfläche 12.

In den Ausführungsformen der Figuren 13 und 14 stellen die Holme 495 und Gelenke 494 bzw. die Holme 625 und Gelenke 624 Tragvorrichtungen für den Bildschirm 491 bzw. für die Röntgenquelle 621 und den Röntgendetektor 622 dar. Aufgrund der Verfahrbarkeit mittels der Laufkatzen 493 bzw. 623 können die Holme 495, 625 wesentlich kürzer sein als herkömmlich und stören deshalb ein vertikales laminares Strömungsfeld in geringerem Maße als herkömmliche Tragarme. Hinzu kommt, dass die Holme 495 und 625 zumindest bei Nichtgebrauch bzw. in einem Ruhezustand des Bildschirms 491 bzw. der Röntgenquelle 621 und des Röntgendetektors 622 vollständig außerhalb des vertikalen laminaren Strömungsfelds angeordnet werden können.

Die Figuren 15 bis 17 zeigen schematische Darstellungen einer Projektionsfläche 41 in einem Betriebszustand 411 (Figur 15) und zwei weiteren Zuständen 418, 419. Die Zeichenebenen der Figuren 15, 16 und 17 entsprechen den Zeichenebenen der Figuren 1 und 4 bis 14 oder schneiden diese in einer vertikalen Geraden. Ähnlich wie bei der oben anhand der Figur 11 dargestellten Tragvorrichtung sind ein Behälter 68 und eine Scherenmechanik 467 vorgesehen. Anstelle eines Gerätehalters ist eine Projektionsfläche, die zwei Bereiche 414, 415 umfasst, vorgesehen, die mittels der Scherenmechanik 467 vertikal verfahrbar bzw. bewegbar ist. Die Projektionsfläche 414, 415 ist kuppelförmig gekrümmt bzw. weist eine positive Gaußsche Krümmung auf. Insbesondere weist die Projektionsfläche 414, 415 die Gestalt eines Ausschnitts einer Kugeloberfläche auf. Die Darstellungen in den Figuren 15 bis 17 zeigen die Projektionsfläche 414, 415 von der Seite, d.h. eine Verbindungsgerade zwischen einem Mittelpunkt der Projektionsfläche 414, 415 und einem Krümmungsmittelpunkt der Projektionsfläche 414, 415 ist parallel zu den Zeichenebenen der Figuren 15 bis 17 und verläuft waagerecht von links nach rechts.

An der dem Betrachter zugewandten Seite ist jeder Bereich 414, 415 der Projektionsfläche mit einer Platte 465, 466 starr verbunden. Die Platte 465 am ersten Bereich 414 der Projektionsfläche und die Platte 466 am zweiten Bereich 415 der Projektionsfläche sind über zwei Verbindungsstäbe 461, 462 gelenkig miteinander verbunden. Die Verbindungsstäbe 461, 462 sind bei dem in den Figuren 15 bis 17 dargestellten Beispiel jeweils bogenförmig, insbesondere in Form eines halben Kreisbogens. Entsprechende Platten und Verbindungsstäbe sind an den in den Figuren 15 bis 17 nicht sichtbaren, vom Betrachter abgewandten Seiten der Bereiche 414, 415 der Projektionsfläche angeordnet.

Die mechanische Kopplung der Bereiche 414, 415 der Projektionsfläche durch die Verbindungsstäbe 461, 462 ermöglicht im Wesentlichen nur eine Bewegung der Bereiche 414, 415 der Projektionsfläche relativ zueinander, bei der beide Bereiche ihre relative Orientierung nicht verändern.

Insbesondere sind die Bereiche 414, 415 der Projektionsfläche von dem in Figur 15 gezeigten Betriebszustand 411 durch eine Bewegung, die in Figur 15 durch kreisbogenförmige Pfeile angedeutet ist, in den in Figur 16 dargestellten Zustand und von diesem durch eine Bewegung, die in Figur 16 durch kreisbogenförmige Pfeile angedeutet ist, in den in Figur 17 gezeigten Ruhezustand überführbar. Es ist erkennbar, dass die vertikale Ausdehnung der Projektionsfläche 414, 415 im Ruhezustand 419 nur etwa halb so groß ist wie im Betriebszustand 411.

Nach der Transformation vom Betriebszustand 411 in den Ruhezustand 419 oder gleichzeitig kann die Projektionsfläche 414, 415 mittels der Scherenmechanik 467 in das in gestrichelten Linien angedeutete Gehäuse 68 bewegt werden. Im Gehäuse 68 kann die Projektionsfläche 414, 415 vor Verschmutzung und Beschädigung geschützt sein, insbesondere wenn abweichend von der Darstellung in den Figuren 15 bis 17 ein Deckel bzw. eine Abdeckung die Öffnung an der Unterseite des Gehäuses 68 verschließt. Aufgrund der Transformation bzw. Überführung der Projektionsfläche 414, 415 in den Ruhezustand 419 kann das Gehäuse 68 deutlich kleiner sein als es sein müsste, wenn es die Projektionsfläche 414, 415 im Betriebszustand 411 aufnehmen müsste. Ein Deckel bzw. eine Abdeckung kann mittels eines Gelenks mit dem Gehäuse 68, insbesondere mit dem Rand der Öffnung im Gehäuse 68 verbunden sein. Alternativ ist ein Deckel bzw. eine Abdeckung starr am ersten Bereich 414 der Projektionsfläche angeordnet, so dass die Öffnung im Gehäuse 68 verschlossen ist, wenn die Projektionsfläche in ihrem Ruhezustand ganz in das Gehäuse 68 eingefahren ist.

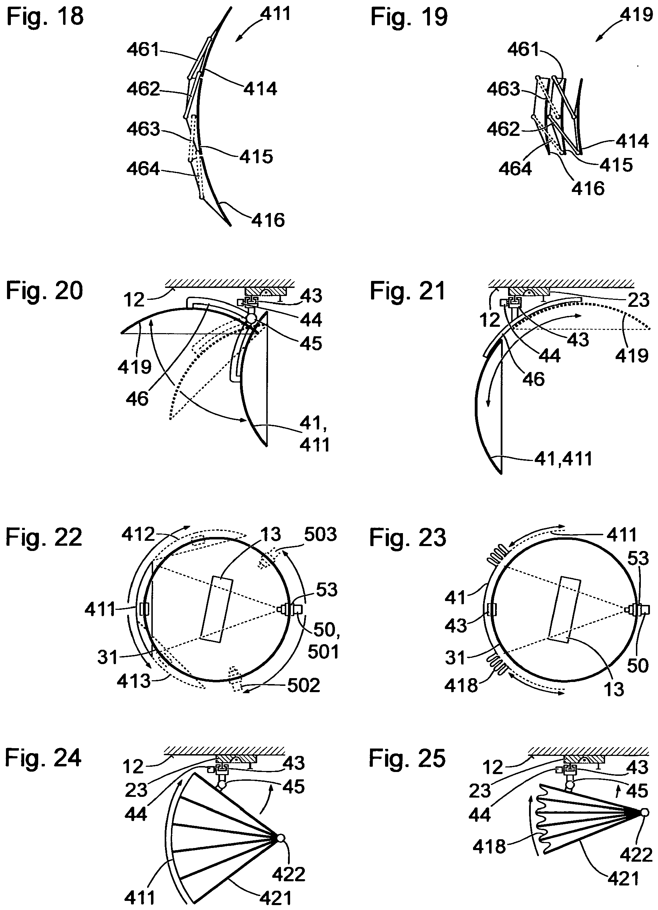

Die Figuren 18 und 19 zeigen schematische Darstellungen einer weiteren verformbaren Projektionsfläche in einem Betriebszustand 411 (Figur 18) und in einem Ruhezustand 419 (Figur 19). Die Zeichenebenen der Figuren 18, 19 entsprechen insbesondere den Zeichenebenen der Figuren 1 und 4 bis 17. Eine entsprechende Transformation ist jedoch alternativ auch in horizontaler Richtung möglich, wobei horizontale Zeichenebenen entsprechende Bilder ergäben.

Die Projektionsfläche weist mehrere Bereiche 414, 415, 416 auf und ist kuppelförmig, insbesondere sphärisch gekrümmt. Die Bereiche 414, 415, 416 der Projektionsfläche sind über Verbindungsstäbe 461, 462, 463, 464 gelenkig miteinander verbunden. Der Abstand der Gelenke zwischen dem ersten Verbindungsstab 461 bzw. dem zweiten Verbindungsstab 462 und dem ersten Bereich 414 der Projektionsfläche ist kleiner als der Abstand der Gelenke zwischen dem ersten Verbindungsstab 461 bzw. dem zweiten Verbindungsstab 462 und dem zweiten Bereich 415 der Projektionsfläche. Alternativ oder zusätzlich weisen die Verbindungsstäbe 461, 462 unterschiedliche Längen zwischen den Gelenken auf. Entsprechendes gilt für den zweiten Bereich 415 der Projektionsfläche und den dritten Bereich 416 der Projektionsfläche sowie den dritten Verbindungsstab 463 und den vierten Verbindungsstab 464. Dadurch bilden die Verbindungsstäbe und die Gelenke an den Bereichen 414, 415, 416 der Projektionsfläche keine Parallelogramme. Dies bewirkt, wie im Vergleich des in Figur 18 gezeigten Betriebszustands 411 und des in Figur 19 gezeigten Ruhezustands 419 erkennbar ist, eine nicht-parallele Verschiebung der Bereiche 414, 415, 416 der Projektionsfläche. Während die Bereiche 414, 415, 416 der Projektionsfläche der Krümmung der Projektionsfläche entsprechend im Betriebszustand 411 unterschiedliche Orientierungen aufweisen, sind sie im Ruhezustand 419 im Wesentlichen parallel zueinander angeordnet. Dies kann ein dichteres Packen der Projektionsfläche bzw. ihrer Bereiche 414, 415, 416 ermöglichen.

Wenn die Projektionsfläche 414, 415, 416 in ihrem in Figur 19 gezeigten Ruhezustand 419 ähnlich wie bei der Ausführungsform der Figuren 15 bis 17 in einem Behälter zu verstauen ist, kann die in Figur 19 erkennbare Parallelität der Bereiche 414, 415, 416 der Projektionsfläche geringere Abmessungen des Behälters ermöglichen.

Bei einer Verformung der Projektionsfläche zwischen ihrem Betriebszustand 411 und ihrem Ruhezustand 419 bzw. der entsprechenden Verschiebung der Bereiche 414, 415, 416 relativ zu einander passiert der Verbindungsstab 462 das Gelenk zwischen dem Verbindungsstab 463 und dem zweiten Bereich 415 der Projektionsfläche. Ferner passiert der Verbindungsstab 463 das Gelenk zwischen dem Verbindungsstab 462 und dem zweiten Bereich 415 der Projektionsfläche. Dies ist möglich, wenn der Verbindungsstab 462 und sein Gelenk zum zweiten Bereich 415 der Projektionsfläche einerseits und der Verbindungsstab 463 und sein Gelenk zum zweiten Bereich 415 der Projektionsfläche andererseits nicht in einer Ebene liegen.

Mit der Darstellung des Verbindungsstabs 462 in durchgezogenen Linien und des Verbindungsstabs 463 in gestrichelten Linien ist angedeutet. dass die Verbindungsstäbe 462, 463 nicht in einer Ebene liegen. Beispielsweise liegen die Ebene, in der der Verbindungsstab 462 (und insbesondere auch der Verbindungsstab 461) schwenkbar ist, und die Ebene, in der der Verbindungsstab 463 (und insbesondere auch der Verbindungsstab 464) schwenkbar ist, parallel und beabstandet zueinander. Allgemeiner müssen die Verbindungsstäbe 462, 463 so angeordnet und geformt sein, dass sie sich nicht gegenseitig blockieren.

Dazu können die Verbindungsstäbe 462, 463 in zwei verschiedenen Ebenen angeordnet oder allgemeiner so angeordnet und ausgebildet sein, dass die von ihnen überstrichenen ebenen oder gekrümmten Flächen einander nicht schneiden. Alternativ können die Verbindungsstäbe 462, 463 jeweils bogenförmig ausgebildet und beispielsweise innerhalb der gleichen Ebene angeordnet sein, ähnlich wie bei dem oben anhand der Figuren 15 bis 17 dargestellten Beispiel.

Figur 20 zeigt eine schematische Darstellung einer weiteren Ausführungsform einer Projektionsfläche 411, die in einigen Merkmalen der oben anhand der Figuren 1 bis 3 dargestellten Projektionsfläche ähnelt. Die Zeichenebene der Figur 21 entspricht insbesondere den Zeichenebenen der Figuren 1 bis 3. Die Projektionsfläche 411 ist ähnlich wie oben anhand der Figuren 1 bis 3 dargestellt mittels einer Laufkatze 43 an einer Schiene 31 verfahrbar befestigt.

Im Unterschied zu der oben anhand der Figuren 1 bis 3 dargestellten Ausführungsform ist ein Gelenk 45 zwischen der Laufkatze 43 und der Projektionsfläche 41 vorgesehen, insbesondere in dem Träger 46. Das Gelenk 45 ermöglicht ein Schwenken der Projektionsfläche 41 um eine horizontale Achse entsprechend den in Figur 2 erkennbaren bogenförmigen Pfeilen zwischen einem Betriebszustand 411 und einem Ruhezustand 419. Im Ruhezustand 419 befindet sich die Projektionsfläche 41 unmittelbar unter der Deckenfläche 12, wo sie medizinisches Personal und seine Bewegungsfreiheit nicht oder in geringerem Maße als im Betriebszustand einschränkt.

Figur 21 zeigt eine schematische Darstellung einer weiteren Projektionsfläche 41, die in einigen Merkmalen den oben anhand der Figuren 1 bis 3 und 20 dargestellten Ausführungsformen ähnelt. Die Zeichenebene der Figur 21 entspricht insbesondere den Zeichenebenen der Figuren 1 bis 3 und 20.

Im Unterschied zu den Ausführungsformen der Figuren 1 bis 3 und 20 ist die Projektionsfläche 41 entlang dem als gekrümmte Schiene bzw. Linearführung ausgebildeten Träger 46 verschiebbar. Aufgrund der Krümmung der Projektionsfläche 41 und der entsprechenden kreisbogenförmigen Krümmung des Trägers 46 kann die Projektionsfläche 41 geführt durch den Träger 46 zwischen einem Betriebszustand 411 und einem Ruhezustand 419 unter der Deckenfläche 12 verschoben bzw. bewegt werden, entsprechend dem in Figur 21 gezeigten bogenförmigen Pfeil. Bei dieser Verschiebung der Projektionsfläche 41 wird diese gleichzeitig um eine horizontale Achse geschwenkt.

Ähnlich wie bei der Ausführungsform der Figur 20 stellt die Projektionsfläche 41 in dem Ruhezustand 419 unter der Deckenfläche 12 kein Hindernis bzw. keine Störung oder Einschränkung für medizinisches Personal und seine Bewegungsfreiheit dar. Wenn das Deckenversorgungssystem 23 und damit die die Laufkatze 43 führende Schiene des Deckenversorgungssystems 23 weit genug von dem in Figur 21 nicht dargestellten vertikalen laminaren Strömungsfeld 21 (vgl. Figur 1) entfernt ist, stellt die Projektionsfläche 41 auch in ihrem Ruhezustand 419 keine Störung für das vertikale laminare Strömungsfeld 21 dar.

Figur 22 zeigt eine weitere schematische Darstellung einer Projektionsfläche, wie sie oben anhand der Figuren 1 bis 3, 20 und 21 beschrieben ist. Die Zeichenebene der Figur 22 entspricht insbesondere den Zeichenebenen der Figuren 2 und 3. Die Projektionsfläche 41 kann in ihrem Betriebszustand entlang des durch die kreisförmige Schiene 31 vorgegebenen Pfads zwischen verschiedenen Betriebspositionen 411, 412, 413 verschoben werden. Dabei kann gleichzeitig der ebenfalls entlang des durch die Schiene vorgegebenen Pfads verschiebbare Projektor 50 in der Projektionsfläche 41 in jeweils diametral gegenüberliegende Arbeitspositionen 501, 502, 503 gebracht werden. Insbesondere werden zumindest entweder die Projektionsfläche 41 abhängig von der Position des Projektors 50 oder der Projektor 50 abhängig von der Position der Projektionsfläche mittels Antriebseinrichtungen und gesteuert durch eine Steuerung so bewegt, das sie einander jederzeit näherungsweise diametral gegenüberliegen.

Figur 23 zeigt eine schematische Darstellung einer weiteren Ausführungsform einer Projektionsfläche 41. Die Zeichenebene der Figur 22 entspricht insbesondere den Zeichenebenen der Figuren 2, 3 und 22. Die Projektionsfläche 41 und ein Projektor 50 sind mittels Laufkatzen 43 bzw. 53 - ähnlich wie oben anhand der Figuren 1 bis 3 und 22 dargestellt - entlang eines durch eine Schiene vorgegebenen Pfads verschiebbar bzw. bewegbar. Bei der Ausführungsform 23 weist die Projektionsfläche 41 in ihrem in Figur 23 nur gestrichelt angedeuteten Betriebszustand die Gestalt eines Ausschnitts eines Kreiszylinders auf. Die Achse des Kreiszylinders ist vertikal bzw. senkrecht zur Zeichenebene der Figur 23, zur Bodenfläche und zur Deckenfläche des medizinischen Behandlungsraums. Die Projektionsfläche 41 kann ähnlich wie ein Vorhang zusammengefaltet werden. Insbesondere kann die Projektionsfläche von ihren seitlichen Rändern her entsprechend den in Figur 23 angedeuteten Pfeilen in einen in Figur 23 in durchgezogener Linie dargestellten Zwischenzustand 418 und weiter in einen in Figur 23 nicht gezeigten vollständig zusammengefalteten Ruhezustand überführt werden. In dem Ruhezustand bildet die Projektionsfläche 41 nur noch ein schmales Bündel, das zusätzlich nach oben zur Deckenfläche hin weggeklappt werden kann, um jede Behinderung des medizinischen Personals und seiner Bewegungsfreiheit zu vermeiden. Die Projektionsfläche 41 kann durch vertikale Holme stabilisiert werden, deren obere Enden beispielsweise an der Schiene 31 oder an einer separaten Schiene, die an der Laufkatze 43 befestigt ist, kreisbogenförmig verschiebbar sind.

Figuren 24 und 25 zeigen schematische Darstellungen einer weiteren Ausführungsform einer Projektionsfläche 41 in einem Betriebszustand 411 (Figur 24) und in einem Zwischenzustand 418 (Figur 25) zwischen dem Betriebszustand 411 und einem nicht dargestellten Ruhezustand. Die Zeichenebenen der Figuren 24 und 25 entsprechen insbesondere den Zeichenebenen der Figuren 1, 4 bis 9, 20 und 21. Die Projektionsfläche 41 ist ähnlich wie bei den Ausführungsformen der Figuren 1 bis 3, 20 und 21 mittels einer Laufkatze 43 an einer Schiene 31 eines Deckenversorgungssystems 23 parallel zu einer Deckenfläche 12 bewegbar befestigt. Im Gegensatz zu den Ausführungsformen der Figuren 1 bis 3, 20 und 21 wird die Projektionsfläche 41 durch eine Oberfläche einer elastischen Membran gebildet, die durch Holme 421 aufgespannt bzw. in eine vorbestimmte Gestalt gebracht werden kann. Jeder einzelne Holm 421 ist kreisbogenförmig, insbesondere halbkreisförmig. Die Enden der Holme 421 sind in zwei einander gegenüberliegenden Gelenken 422 zusammengefasst. In dem in Figur 24 gezeigten Betriebszustand 411 der Projektionsfläche 41 weist die Projektionsfläche zwischen zwei benachbarten Holmen 421 jeweils näherungsweise die Gestalt eines Kreiszylinders auf. Insgesamt weist die Projektionsfläche 41 damit näherungsweise die Gestalt eines Ausschnitts einer Kugeloberfläche auf.

Ausgehend von dem in Figur 24 gezeigten Betriebszustand 411 der Projektionsfläche 41 können die Holme dem in Figur 24 links gezeigten Pfeil entsprechend zusammengeklappt und dem in Figur 24 rechts gezeigte Pfeil entsprechend nach oben geschwenkt werden. Über den in Figur 25 gezeigten Zwischenzustand 418 kann die Projektionsfläche so in einen nicht dargestellten Ruhezustand überführt bzw. transformiert bzw. verformt werden. In diesem Ruhezustand bildet die Projektionsfläche 41 ein schmales bogenförmiges Bündel, das unter der Deckenfläche 12 angeordnet ist. In diesem hier nicht dargestellten Ruhezustand nimmt die Projektionsfläche nur einen geringen Raum ein und stellt keine Einschränkung für das medizinische Personal und seine Bewegungsfreiheit dar.

Figur 26 zeigt eine schematische Darstellung eines Projektionssystems alternativ zu den in den Figuren 1 bis 3, 22 und 23 gezeigten Projektoren. Die Zeichenebene der Figur 26 ist insbesondere parallel zu den Zeichenebenen der Figur 1. Das in Figur 26 gezeigte Projektionssystem kann in der Mitte des von den Luftdüsen 21 eingenommenen Bereichs der Deckenfläche und insbesondere in der Mitte eines Kreises, einer Ellipse oder eines Ovals, das durch ein Deckenversorgungssystem oder eine oder mehrere Schienen parallel zur Deckenfläche 12 gebildet wird, angeordnet sein. Durch diese Anordnung muss bei einer Bewegung einer Projektionsfläche entlang eines durch die Schiene vorgegebenen Pfads das Projektionssystem nicht verschoben, sondern lediglich um eine vertikale Achse 59 rotiert werden. Um ein vertikales laminares Strömungsfeld 22 möglichst wenig zu stören, ist das Projektionssystem zumindest teilweise über der Deckenfläche 12 angeordnet.

Insbesondere umfasst das Projektionssystem eine Lichtquelle 51, einen Kollimator 52 und einen ersten Spiegel 551, die über der Deckenfläche 12 angeordnet sind, und einen zweiten Spiegel 552, einen Lichtmodulator 57 und eine Abbildungseinrichtung 58, die unter der Deckenfläche 12 angeordnet sind. Die Abbildungseinrichtung 58 ist insbesondere eine Linse, ein Objektiv oder ein Spiegelsystem, das abbildende Eigenschaften aufweist. Der Kollimator 52 kollimiert das von der Lichtquelle 51 erzeugte Licht. Das weitgehend parallele Licht wird über die beiden Spiegel 551, 552 auf den Lichtmodulator 57 gelenkt. Der Lichtmodulator 57 ist beispielsweise ein LCD-Bauelement. Das vom Lichtmodulator 57 erzeugte Bild wird von der Abbildungseinrichtung 58 auf eine Projektionsfläche 41 abgebildet. Der Kollimator 52 und die Spiegel 551, 552 sowie ein Tubus 553 zwischen diesen definieren den Querschnitt eines Strahlengangs 55 zum Übertragen bzw. Leiten von Licht der Lichtquelle 51 zu dem Lichtmodulator 57.

Durch Rotation des zweiten Spiegels 552, des Lichtmodulators 57 und der Abbildungseinrichtung 58 um eine vertikale Schwenkachse 59 kann die Richtung, in der ein Bild projiziert wird, an den Ort der Projektionsfläche 41 angepasst werden. Durch Kippen und Verschieben des Lichtmodulators 57, der Abbildungseinrichtung 58 und des zweiten Spiegels 552 sowie einzelner, in Figur 26 nicht dargestellter Komponenten (insbesondere Linsen und Linsengruppen) der Abbildungseinrichtung 58 sind eine Anpassung der Projektionsrichtung sowie der Lage und der räumlichen Gestalt des von der Abbildungseinrichtung 58 erzeugten Bilds an den Ort, die Orientierung und die Gestalt der Projektionsfläche 41 möglich. Ferner kann das von der Abbildungseinrichtung 58 erzeugte Bild des Lichtmodulators 57 durch Verschieben und Kippen des Lichtmodulators 57 und der Abbildungseinrichtung 58 abhängig von der Position eines Betrachters so verformt werden, dass der Betrachter das Bild auf der Projektionsfläche 41 unverzerrt wahrnimmt, insbesondere parallele Geraden in dem Bild als parallel und rechte Winkel als rechte Winkel.

Abweichend von der Darstellung in Figur 26 können der Lichtmodulator 57 oder der Lichtmodulator 57 und zumindest ein Teil der Abbildungseinrichtung 58 über der Deckenfläche 12 angeordnet sein. Dadurch können die Abmessung der unter der Deckenfläche 12 angeordneten Komponenten des Projektionssystems und die dadurch hervorgerufenen Störungen des vertikalen laminaren Strömungsfelds 22 weiter reduziert werden.

Wenn der Lichtmodulator 57 und die Abbildungseinrichtung 58 anders als in Figur 26 dargestellt nicht gemeinsam um die vertikale Schwenkachse 59 schwenkbar sind, kann eine Rotation der Abbildungseinrichtung 58 um die vertikale Schwenkachse 59 ein Kippen des projizierten Bilds bewirken. Dies gilt beispielsweise, wenn der Lichtmodulator 57 über der Deckenfläche 12 feststehend angeordnet ist. Zur Kompensation kann eine zur Rotation der Abbildungseinrichtung 58 um die vertikale Schwenkachse 59 simultane Rotation des Lichtmodulators um die lokale optische Achse des Projektionssystems und insbesondere um seine Flächennormale vorgesehen sein. Dazu kann eine entsprechende mechanische Kopplung zwischen der Abbildungseinrichtung 58 und dem Lichtmodulator 57 oder eine entsprechende logische Kopplung mit einer motorischen Steuerung, die beide Bewegungen koppelt, vorgesehen sein.

Alternativ kann zwischen dem feststehenden Lichtmodulator 57 und der um die vertikale Schwenkachse 59 schwenkbaren Abbildungseinrichtung 58 eine Einrichtung zum Aufrichten eines Bilds vorgesehen sein, beispielsweise eine Anordnung von einem oder mehreren Prismen. Diese Einrichtung zum Aufrichten des Bilds wird durch eine mechanische oder logische Kopplung simultan zu einem Schwenken der Abbildungseinrichtung 58 um die vertikale Schwenkachse 59 rotiert, wobei die Winkelgeschwindigkeiten in einem festen Verhältnis zueinander stehen.

Alternativ kann das Projektionssystem oder eine den Lichtmodulator 57 steuernde Einrichtung ausgebildet sein, um das am Lichtmodulator 57 entstehende Modulationsmuster entsprechend einer Schwenkbewegung der Abbildungseinrichtung 58 um die vertikale Schwenkachse 59 zu rotieren.

Figur 27 zeigt eine schematische Darstellung eines weiteren Projektionssystems. Die Zeichenebene der Figur 27 entspricht der Zeichenebene der Figur 26. Eine Abbildungseinrichtung 58 ist ähnlich wie bei der Ausführungsform der Figur 26 unter der Deckenfläche 12 angeordnet, eine Lichtquelle 51 ist über der Deckenfläche 12 angeordnet. Licht von der Lichtquelle 51 wird jedoch nicht mittels Spiegeln, sondern mittels eines Lichtleitkabels 557 von der Lichtquelle 51 über der Deckenfläche 12 zu einem Lichtmodulator 57 und der Abbildungseinrichtung 58 übertragen. Zur Einkopplung des von der Lichtquelle 51 erzeugten Lichts in das Lichtleitkabel 557 ist insbesondere eine Sammellinse 52 vorgesehen. Das aus dem Lichtleitkabel 557 ausgekoppelt Licht wird von einem Kollimator 56 kollimiert und dann vom Lichtmodulator 57 moduliert. Der Lichtmodulator wird von der Abbildungseinrichtung 58 auf eine in Figur 27 nicht dargestellte Projektionsfläche abgebildet.

Ähnlich wie oben anhand der Figur 26 dargestellt, ist durch Rotation des Kollimators 56, des Lichtmodulators 57 und der Abbildungseinrichtung 58 um eine vertikale Schwenkachse 59 und Schwenken um eine in Figur 27 nicht gezeigte horizontale Achse sowie durch Kippen und Verschieben des Lichtmodulators 57 und der Abbildungseinrichtung 58 oder von Komponenten der Abbildungseinrichtung 58 relativ zueinander eine Einstellung der Projektionsrichtung, der Lage und der Gestalt der Projektionsfläche möglich. Ferner ist eine Verformung des Bilds zur Erzeugung eines unverzerrten Eindrucks bei einem Betrachter möglich. Letzteres ist bei beiden Ausführungsformen der Figuren 26 und 27 alternativ oder zusätzlich durch Veränderung des den Lichtmodulator 57 steuernden Signals möglich.

Bei den meisten der oben anhand der Figuren 1 bis 3 und 15 bis 25 dargestellten Ausführungsbeispielen können anstelle von Projektionsflächen 41 Bildflächen vorgesehen sein, die beispielsweise als starre oder flexible OLED-Displays ausgebildet sind, um Licht zu emittieren und ohne einen Projektor zu erfordern Information darzustellen. Sofern die Bildfläche nicht wie beispielsweise bei den Ausführungsbeispielen der Figuren 23 bis 25 elastisch ist, kann die Bildfläche alternativ eine starre Flüssigkristallanzeige (LCD-Display) einen starren Plasmabildschirm oder einen anderen starren Bildschirm umfassen.

Figur 28 zeigt eine schematische Darstellung einer Steuerung 80 zum Steuern von einer oder mehreren Funktionen der Systeme, Vorrichtungen und Einrichtungen, die oben anhand der Figuren 1 bis 27 dargestellt wurden. Die Steuerung ist insbesondere mit der anhand der Figur 1 dargestellten Raumkamera 24 und weiteren Raumkameras 241, 242, einem externen Bildspeicher 85, einem Endoskopiesystem 69 mit einem Endoskop 691 und einem ortsempfindlichen Röntgendetektor bzw. röntgenempfindlichen Bildsensor 622 gekoppelt, um von diesen Bilder zu empfangen. Ferner kann die Steuerung 80 mit den Raumkameras 24, 241, 242, dem Endoskopiesystem 69, 691, dem externen Bildspeicher 85, einer Röntgenquelle 621 und dem röntgenempfindlichen Bildsensor 622 gekoppelt sein, um deren Betriebsparameter einzustellen, sie zu bewegen, zu schwenken und zu steuern.

Ferner ist die Steuerung 80 mit einem oder mehreren Projektoren 50 gekoppelt, um Bildsignale an die Projektoren 50 zu senden. Ferner ist die Steuerung 80 mit Antriebseinrichtungen 44, 54, 64 an Projektionsflächen, Projektoren und Tragvorrichtungen gekoppelt, um Bewegungen von Laufkatzen entlang einer oder mehreren Schienen sowie Translationsbewegungen, insbesondere vertikale Translationsbewegungen, Schwenk- und Rotationsbewegungen zu steuern. Dadurch bilden die Antriebseinrichtungen 44, 54, 64 und die Steuerung 80 Positionierungseinrichtungen zur Positionierung von Projektionsflächen, Projektoren, Tragvorrichtungen oder medizinischen Geräten.

Die Steuerung umfasst eine interne Bildquelle 81, eine insbesondere mit den Raumkameras 24, 241, 242 oder anderen Sensoren gekoppelte Gestenerkennung 82, eine Positionierung 83 und eine Entzerrung 84. Die Gestenerkennung 82, die Positionierung 83 und die Entzerrung 84 können jeweils teilweise oder vollständig durch separate analoge oder digitale elektronische Schaltungen oder teilweise oder vollständig durch Software oder Firmware realisiert sein.

Die Steuerung 80 ist insbesondere ausgebildet, um eine oder mehrere Projektionsflächen und eine oder mehrere Projektoren oder Projektionssysteme abhängig von Gesten medizinischen Personals und/oder abhängig von einer Phase in einem Arbeitsablauf und/oder abhängig von einer Position oder einer Blickrichtung medizinischen Personals so einzustellen, dass das medizinische Personal unter ergonomisch günstigen Bedingungen arbeiten kann. Ferner ist die Steuerung 80 ausgebildet, um Projektionsflächen, Projektoren, Kameras, Tragvorrichtungen und andere motorisch bewegbare Einrichtungen so zu bewegen bzw. zu konfigurieren, dass sie sich gegenseitig und das medizinische Personal nicht behindern oder beschädigen, und dass beispielsweise ein Projektor und eine Projektionsfläche im Wesentlichen einander gegenüber positioniert sind.

### Bezugszeichen

- 10: medizinischer Behandlungsraum
- 11: Bodenfläche des medizinischen Behandlungsraums 10
- 12: Deckenfläche des medizinischen Behandlungsraums 10
- 13: Behandlungstisch
- 14: Patient auf dem Behandlungstisch
- 15: Operationsfeld
- 16: medizinisches Personal
- 17: Kopf des medizinischen Personals 16
- 18: Hand des medizinischen Personals 16
- 21: Luftdüse
- 22: Strömungsfeld
- 23: Deckenversorgungssystem
- 24: Raumkamera
- 241: weitere Raumkamera
- 242: weitere Raumkamera
- 25: Allgemeinbeleuchtung
- 26: Operationsleuchte
- 28: Blende
- 29: Kontaktschiene
- 31: Schiene
- 311: Führungssteg an der Schiene 31
- 32: Schiene
- 321: Führungssteg an der Schiene 32
- 33: Schiene
- 34: Schiene
- 37: durch Schiene 31 vorgegebener Pfad
- 39: durch Schiene 33 vorgegebener zweiter Pfad
- 41: Projektionsfläche
- 411: Projektionsfläche 41 in erstem Betriebszustand
- 412: Projektionsfläche 41 in zweitem Betriebszustand
- 413: Projektionsfläche 41 in drittem Betriebszustand
- 414: erster Bereich der Projektionsfläche 41
- 415: zweiter Bereich der Projektionsfläche 41
- 416: dritter Bereich der Projektionsfläche 41
- 418: Projektionsfläche 41 in Zustand zwischen Betriebszustand und Ruhezustand
- 419: Projektionsfläche 41 in Ruhezustand
- 421: Holm
- 43: Laufkatze der Projektionsfläche 40
- 430: Laufrolle an der Laufkatze 43
- 435: Gleitbelag an der Laufkatze 43
- 439: Schleifkontakt (Kontakteinrichtung) an der Laufkatze 43
- 44: Antriebseinrichtung der Laufkatze 43 der Projektionsfläche 40
- 45: Gelenk
- 46: Träger für die Projektionsfläche 41
- 461: erster Verbindungsstab zwischen Bereichen der Projektionsfläche 41
- 462: zweiter Verbindungsstab zwischen Bereichen der Projektionsfläche 41
- 463: dritter Verbindungsstab zwischen Bereichen der Projektionsfläche 41
- 464: vierter Verbindungsstab zwischen Bereichen der Projektionsfläche 41
- 465: Platte am ersten Bereich 414 der Projektionsfläche
- 466: Platte am zweiten Bereich 415 der Projektionsfläche
- 467: Scherenmechanik
- 48: Kugeloberfläche
- 490: Anzeigevorrichtung
- 491: Bildschirm
- 493: Laufkatze der Anzeigevorrichtung 49
- 494: Gelenk
- 495: Holm
- 496: Schwenkachse
- 50: Projektor bzw. Projektionssystem
- 51: Lichtquelle
- 52: Kollimator oder Sammellinse
- 53: Laufkatze des Projektors 50
- 530: Laufrolle an der Laufkatze 53
- 54: Antriebseinrichtung der Laufkatze 53 des Projektors 50
- 55: Strahlengang
- 551: erster Umlenkspiegel
- 552: zweiter Umlenkspiegel
- 553: Tubus
- 557: Lichtleitkabel
- 56: Kollimator
- 57: Lichtmodulator
- 58: Abbildungseinrichtung
- 59: Schwenkachse der Abbildungseinrichtung 58
- 60: Tragvorrichtung
- 621: Röntgenquelle
- 622: ortsempfindlicher Röntgendetektor
- 623: Laufkatze der Anzeigevorrichtung 49
- 624: Gelenk
- 625: Holm
- 626: Schwenkachse
- 627: C-förmiger Bogen
- 63: Laufkatze der Tragvorrichtung 60
- 64: Antriebsvorrichtung der Laufkatze 63 der Tragvorrichtung 60
- 651: dehnbare Leitung zwischen dem Behälter 68 und dem Gerätehalter 67
- 652: aufrollbare Leitung zwischen dem Behälter 68 und dem Gerätehalter 67
- 653: Haspel zum Aufrollen der aufrollbaren Leitung 652
- 66: vertikaler Holm der Tragvorrichtung 60
- 661: Seil
- 663: Haspel
- 664: Antriebseinrichtung für Haspel 663
- 667: Scherenmechanik
- 67: Gerätehalter der Tragvorrichtung 60
- 671: Betriebsposition des Gerätehalters 67
- 679: Ruheposition des Gerätehalters 67
- 68: Behälter der Tragvorrichtung 60
- 680: Öffnung im Behälter 68
- 687: Öffnung im Behälter 68
- 69: medizinisches Gerät
- 71: Versorgungsarm
- 72: Gelenk am radial inneren Ende des Versorgungsarms 71
- 73: radial äußeres Ende des Versorgungsarms 71
- 74: Führungsschiene für radial äußeres Ende 73 des Versorgungsarms 71
- 75: elastische Leitung
- 761: Bereitstellungsarm
- 762: Holm
- 763: Gelenk
- 765: Leitung
- 768: Satelliteneinrichtung
- 769: Anschluss
- 80: Steuerung
- 81: interne Bildquelle
- 82: Gestenerkennung
- 83: Positionierung
- 84: Entzerrung
- 85: externe Bildquelle

## Patentansprüche

1. **Tragesystem** für einen medizinischen Behandlungsraum (10), mit:
einer an einer Decke (12) des medizinischen Behandlungsraums (10) zu befestigenden **Schieneneinrichtung** (23, 31, 32, 33, 34), die einen gekrümmten Pfad (37, 39) vorgibt,
einer **Hubeinrichtung** (661, 663, 664; 667) zum Bewegen eines medizinischen Geräts (41, 50; 69) zwischen einer oberen Ruheposition (679) und einer unteren Betriebsposition (671),
wobei die Hubeinrichtung (661, 663, 664; 667) entlang des von der Schieneneinrichtung (23, 31, 32, 33, 34) vorgegebenen Pfads (37, 39) **bewegbar** ist **gekennzeichnet durch** einen **Versorgungsarm** (71), der um einen durch ein Gelenk (72) vorbestimmten Ort rotierbar ist, wobei der Versorgungsarm (71) an einem von dem Gelenk (72) beabstandeten Ort mit der Hubeinrichtung (661, 663, 664; 667) oder mit der Tragvorrichtung (60) gekoppelt ist, und wobei der Versorgungsarm (71) zur Übertragung zumindest entweder eines Signals oder von Leistung oder eines Fluids ausgebildet ist.

2. Tragesystem nach dem vorangehenden Anspruch, bei der das medizinische Gerät eine Anzeigevorrichtung (41, 50) oder ein Teil (41; 50) einer Anzeigevorrichtung ist, wobei mittels der Anzeigevorrichtung (41, 50) in der unteren Betriebsposition (671) Information für medizinisches Personal sichtbar dargestellt werden kann,

3. Tragesystem nach dem vorangehenden Anspruch, bei dem die Anzeigevorrichtung eine **Projektionsfläche** (41), einen **Projektor** (50) zum Projizieren einer Darstellung von Information auf die Projektionsfläche (40) und eine **Positionierungseinrichtung** (44, 54, 80) zur Festlegung der Positionen des Projektors (50) und der Projektionsfläche (41) umfasst, wobei die Positionierungseinrichtung (44, 54, 80) ausgebildet ist, um den Projektor (50) und die Projektionsfläche (41) an im Wesentlichen **gegenüberliegenden Positionen** des von der Schieneneinrichtung (23, 31, 32, 33, 34) vorgegebenen Pfads (37, 39) zu positionieren.

4. Tragesystem nach dem vorangehenden Anspruch, bei dem die Positionierungseinrichtung eine Steuerung (80) und zumindest entweder eine **erste Antriebseinrichtung** (54) zum Bewegen des Projektors (50) entlang des durch die Schieneneinrichtung (23, 31, 32, 33, 34) vorgegebenen Pfads (37, 39) oder eine **zweite Antriebseinrichtung** (44) zum Bewegen der Projektionsfläche (41) entlang des durch die Schieneneinrichtung (23, 31, 32, 33, 34) vorgegebenen Pfads (37, 39), wobei die Steuerung (80) einen Steuerausgang zum Steuern zumindest entweder der ersten Antriebseinrichtung (44) oder der zweiten Antriebseinrichtung (54) aufweist.

5. Tragesystem nach dem vorangehenden Anspruch, bei dem die Steuerung (80) einen **Eingang** zum Empfangen eines **Positionssignals,** das von der Position der Projektionsfläche (41) relativ zum Projektor (50) oder von den absoluten Positionen von Projektionsfläche (41) und Projektor (50) abhängig ist, umfasst.

6. Tragesystem nach einem der vorangehenden Ansprüche, bei dem der von der Schieneneinrichtung (23, 31, 32, 33, 34) vorgegebene Pfad (37, 39) insbesondere die Gestalt eines Teils einer Randlinie oder einer vollständigen Randlinie einer Ellipse oder eines Kreises oder eines Rechtecks mit abgerundeten Ecken oder einer Parabel oder einer Hyperbel aufweist.

7. Tragesystem nach einem der vorangehenden Ansprüche, ferner mit:
einer Übertragungseinrichtung (29, 439) zum elektrischen Übertragen zumindest entweder von elektrischer Leistung oder eines elektrischen Signals, wobei die Übertragungseinrichtung eine starr angeordnete **Kontaktschiene** (29) und eine mit der Hubeinrichtung (661, 663, 664; 667) mechanische gekoppelte und **bewegbare Kontakteinrichtung** (439), die mit der Kontaktschiene (29) einen mechanischen und elektrisch leitfähigen Kontakt bildet, umfasst.

8. Tragesystem nach einem der vorangehenden Ansprüche, ferner mit:
einer **Tragvorrichtung** (60) zum Halten eines weiteren medizinischen Geräts (69), wobei die Tragvorrichtung (60) entlang des von der Schieneneinrichtung (23, 31, 32, 33, 34) vorgegebenen Pfads (37, 39) **bewegbar** ist.

9. Tragesystem nach dem vorangehenden Anspruch, bei dem die Schieneneinrichtung mehrere Schienen (31, 32, 33, 34) umfasst, wobei die Tragvorrichtung (60) an einer Schiene (31, 32, 33, 34) hängt, an der die Hubeinrichtung (661, 663, 664, 667) nicht hängt.

10. Tragesystem nach dem vorangehenden Anspruch, bei dem der Versorgungsarm (71) **nicht** zur Aufnahme der **Gewichtskraft** der Hubeinrichtung (661, 663, 664; 667) oder der Tragvorrichtung (60) oder eines medizinischen Geräts (4, 50; 69) ausgebildet ist.

11. Tragesystem nach einem der vorangehenden Ansprüche, ferner mit einer Führungseinrichtung (66, 667) zum Führen des medizinischen Geräts (41, 50, 69) entlang eines vorbestimmten Pfads (37, 39) zwischen der oberen Ruheposition (679) und der unteren Betriebsposition (671).

12. Tragesystem nach dem vorangehenden Anspruch, bei der die Führungseinrichtung eine **Scherenmechanik** (467, 667) umfasst, die ausgebildet ist, um das medizinische Gerät (41, 50, 69) unabhängig von seiner Position (671, 679) in einer vorbestimmten Orientierung zu halten.

13. Tragesystem nach Anspruch 11, bei der die Führungseinrichtung eine **teleskopierbare** Einrichtung (66) umfasst.

14. Tragesystem nach einem der vorangehenden Ansprüche, ferner mit:
einem Seil (661) und eine Antriebseinrichtung (663, 664) zum Bewegen des medizinischen Geräts (41, 50, 69) zwischen der Ruheposition (679) und der Betriebsposition (671) mittels des Seils (661).

## Claims

1. Carrying system for a medical treatment room (10), comprising:
a track device (23, 31, 32, 33, 34) which is to be secured on a ceiling (12) of the medical treatment room (10) and which predefines a curved path (37, 39),
a lifting device (661, 663, 664; 667) for moving a medical appliance (41, 50; 69) between an upper rest position (679) and a lower operating position (671),
wherein the lifting device (661, 663, 664; 667) is movable along the path (37, 39) predefined by the track device (23, 31, 32, 33, 34), **characterized by** a supply arm (71) which is rotatable about a location predetermined by a joint (72), wherein the supply arm (71) is coupled to the lifting device (661, 663, 664; 667) or to the carrying device (60) at a location spaced apart from the joint (72), and wherein the supply arm (71) is configured to transmit at least either a signal or power or a fluid.

2. Carrying system according to the preceding claim, in which the medical appliance is a display apparatus (41, 50) or a part (41; 50) of a display apparatus, wherein information for medical personnel can be presented visually by means of the display apparatus (41, 50) in the lower operating position (671).

3. Carrying system according to the preceding claim, in which the display apparatus comprises a projection surface (41), a projector (50) for projecting a presentation of information onto the projection surface (40), and a positioning device (44, 54, 80) for establishing the positions of the projector (50) and of the projection surface (41), wherein the positioning device (44, 54, 80) is configured to position the projector (50) and the projection surface (41) at substantially opposite positions of the path (37, 39) predefined by the track device (23, 31, 32, 33, 34).

4. Carrying system according to the preceding claim, in which the positioning device comprises a control (80) and at least either a first drive device (54) for moving the projector (50) along the path (37, 39) predefined by the track device (23, 31, 32, 33, 34) or a second drive device (44) for moving the projection surface (41) along the path (37, 39) predefined by the track device (23, 31, 32, 33, 34), wherein the control (80) has a control output for controlling at least either the first drive device (44) or the second drive device (54) .

5. Carrying system according to the preceding claim, in which the control (80) comprises an input for receiving a position signal that is dependent on the position of the projection surface (41) relative to the projector (50) or on the absolute positions of projection surface (41) and projector (50) .

6. Carrying system according to one of the preceding claims, in which the path (37, 39) predefined by the track device (23, 31, 32, 33, 34) has in particular the shape of part of an edge line or a complete edge line of an ellipse or of a circle or of a rectangle with rounded corners or of a parabola or a hyperbola.

7. Carrying system according to one of the preceding claims, further comprising:
a transmission device (29, 439) for electrically transmitting at least either electrical power or an electrical signal, wherein the transmission device comprises a rigidly arranged contact track (29) and a movable contact device (439) which is mechanically coupled to the lifting device (661, 663, 664; 667) and which, with the contact track (29), forms a mechanical and electrically conductive contact.

8. Carrying system according to one of the preceding claims, further comprising:
a carrying device (60) for holding a further medical appliance (69), wherein the carrying device (60) is movable along the path (37, 39) predefined by the track device (23, 31, 32, 33, 34) .

9. Carrying system according to the preceding claim, in which the track device comprises several tracks (31, 32, 33, 34), wherein the carrying device (60) is suspended from a track (31, 32, 33, 34) from which the lifting device (661, 663, 664, 667) is not suspended.

10. Carrying system according to the preceding claim, in which the supply arm (71) is not configured to take up the weight of the lifting device (661, 663, 664; 667) or of the carrying device (60) or of a medical appliance (4, 50; 69).

11. Carrying system according to one of the preceding claims, further comprising a guide device (66, 667) for guiding the medical appliance (41, 50, 69) device along a predetermined path (37, 39) between the upper rest position (679) and the lower operating position (671).

12. Carrying system according to the preceding claim, in which the guide device comprises a scissor mechanism (467, 667), which is configured to hold the medical appliance (41, 50, 69) in a predetermined orientation independently of its position (671, 679).

13. Carrying system according to Claim 11, in which the guide device comprises a telescopic device (66) .

14. Carrying system according to one of the preceding claims, further comprising:
a cable (661) and a drive device (663, 664) for moving the medical appliance (41, 50, 69) between the rest position (679) and the operating position (671) by means of the cable (661).

## Revendications

1. Système de support pour une salle de traitement médicale (10), avec:
un système de rails (23, 31, 32, 33, 34) à fixer à un plafond (12) de la salle de traitement médicale (10), qui définit un chemin courbe (37, 39),
un système de levage (661, 663, 664; 667) pour le déplacement d'un appareil médical (41, 50; 69) entre une position de repos supérieure (679) et une position de fonctionnement inférieure (671),
dans lequel le système de levage (661, 663, 664; 667) est déplaçable le long du chemin (37, 39) défini par le système de rails (23, 31, 32, 33, 34),
**caractérisé par** un bras d'alimentation (71), qui peut tourner autour d'un point prédéterminé par une articulation (72), dans lequel le bras d'alimentation (71) est couplé en un point distant de l'articulation (72) au système de levage (661, 663, 664; 667) ou au dispositif de support (60), et dans lequel le bras d'alimentation (71) est conçu pour la transmission d'au moins soit un signal soit une puissance soit un fluide.

2. Système de support selon la revendication précédente, dans lequel l'appareil médical est un dispositif d'affichage (41, 50) ou une partie (41 ; 50) d'un dispositif d'affichage, dans lequel des informations destinées au personnel médical peuvent être représentées d'une façon visible au moyen du dispositif d'affichage (41, 50) dans la position de fonctionnement inférieure (671).

3. Système de support selon la revendication précédente, dans lequel le dispositif d'affichage comprend une surface de projection (41), un projecteur (50) pour la projection d'une représentation d'informations sur la surface de projection (40) et un système de positionnement (44, 54, 80) pour la fixation de la position du projecteur (50) et de la surface de projection (41), dans lequel le système de positionnement (44, 54, 80) est conçu pour positionner le projecteur (50) et la surface de projection (41) en des positions se faisant essentiellement face du chemin (37, 39) prédéterminé par le système de rails (23, 31, 32, 33, 34) .

4. Système de support selon la revendication précédente, dans lequel le système de positionnement présente une commande (80) et au moins soit un premier dispositif d'entraînement (54) pour déplacer le projecteur (50) le long du chemin (37, 39) prédéterminé par le système de rails (23, 31, 32, 33, 34) soit un deuxième dispositif d'entraînement (44) pour déplacer la surface de projection (41) le long du chemin (37, 39) prédéterminé par le système de rails (23, 31, 32, 33, 34), dans lequel la commande (80) présente une sortie de commande pour commander au moins soit le premier dispositif d'entraînement (44) soit le deuxième dispositif d'entraînement (54).

5. Système de support selon la revendication précédente, dans lequel la commande (80) comprend une entrée pour recevoir un signal de position, qui dépend de la position de la surface de projection (41) par rapport au projecteur (50) ou des positions absolues de la surface de projection (41) et du projecteur (50).

6. Système de support selon l'une quelconque des revendications précédentes, dans lequel le chemin (37, 39) prédéterminé par le système de rails (23, 31, 32, 33, 34) présente en particulier la forme d'une partie d'un périmètre ou d'un périmètre complet d'une ellipse ou d'un cercle ou d'un rectangle avec des coins arrondis ou d'une parabole ou d'une hyperbole.

7. Système de support selon l'une quelconque des revendications précédentes, avec en outre:
un système de transmission (29, 439) pour la transmission électrique d'au moins soit une puissance électrique soit un signal électrique, dans lequel le système de transmission comprend un rail de contact (29) disposé de façon fixe et un système de contact mobile (439) et couplé mécaniquement au système de levage (661, 663, 664; 667), qui forme avec le rail de contact (29) un contact mécanique et électriquement conducteur.

8. Système de support selon l'une quelconque des revendications précédentes, avec en outre:
un dispositif de support (60) pour maintenir un autre appareil médical (69), dans lequel le dispositif de support (60) est mobile le long du chemin (37, 39) prédéterminé par le système de rails (23, 31, 32, 33, 34) .

9. Système de support selon la revendication précédente, dans lequel le système de rails comprend plusieurs rails (31, 32, 33, 34), dans lequel le dispositif de support (60) est suspendu à un rail (31, 32, 33, 34), auquel le système de levage (661, 663, 664; 667) n'est pas suspendu.

10. Système de support selon la revendication précédente, dans lequel le bras d'alimentation (71) n'est pas configuré pour reprendre le poids du système de levage (661, 663, 664; 667) ou du dispositif de support (60) ou de l'appareil médical (41, 50; 69).

11. Système de support selon l'une quelconque des revendications précédentes, avec en outre un système de guidage (66, 667) pour le guidage de l'appareil médical (41, 50, 69) le long d'un chemin prédéterminé (37, 39) entre la position de repos supérieure (679) et la position de fonctionnement inférieure (671).

12. Système de support selon la revendication précédente, dans lequel le système de guidage comprend un mécanisme à ciseaux (467, 667), qui est conçu pour maintenir l'appareil médical (41, 50, 69) dans une orientation prédéterminée indépendamment de sa position (671, 679).

13. Système de support selon la revendication 11, dans lequel le système de guidage comprend un système télescopique (66).

14. Système de support selon l'une quelconque des revendications précédentes, avec en outre:
un câble (661) et un système d'entraînement (663, 664) pour déplacer l'appareil médical (41, 50, 69) entre la position de repos (679) et la position de fonctionnement (671) au moyen du câble (661).
